# EUROPEAN PATENT APPLICATION

(11) **EP 4 495 138 A1**
(43) Date of publication of application: **22.01.2025**
(21) Application number: 22931427.3
(22) Date of filing: 18.03.2022
(51) Int. Cl.: C07K 16/28, C07K 16/46, C07K 19/00, C12N 7/01, C12N 15/13, A61K 39/395, A61P 35/00, A61P 37/04

(54) **B7-H3-BINDING ANTIBODY AND USE THEREOF**

(71) Applicant: Beijing Mabworks Biotech Co., Ltd, Beijing 100176 (CN)
(72) Inventor: LI, Jiangmei, Beijing 100176 (CN); CHEN, Yunyun, Beijing 102600 (CN); MAO, Ting, Beijing 102600 (CN); GUO, Yanan, Beijing 102600 (CN); HU, Wenqi, Beijing 100176 (CN); LI, Feng, Beijing 100176 (CN)
(74) Representative: Krauss, Jan
(86) International application number: PCT/CN2022/081615
(87) International publication number: WO 2023/173393

(57) **Abstract**

The application provides an isolated monoclonal antibody or an antigen binding portion thereof, that specifically binds to B7-H3. Also provided is a nucleic acid molecule that encodes the antibody or antigen binding portion thereof, and an expression vector, a host cell and a method for expressing the antibody or antigen binding portion thereof. The application further provides an immunoconjugate, a bispecific molecule, a chimeric antigen receptor, an oncolytic virus, and a pharmaceutical composition that comprise the antibody or antigen binding portion thereof, and a treatment method using the antibody or antigen binding portion thereof of the disclosure.

## Description

### FIELD OF THE INVENTION

The present application relates to an antibody specifically binding human B7-H3, and its preparation and uses, especially its use in treating B7-H3 associated diseases such as cancers.

### BACKGROUND OF THE INVENTION

T cell proliferation and activation rely on dual signals. The interaction between the T cell receptor with the peptide presented by the MHC on an antigen-presenting cell or a tumor cell provides the first signal which is essential for T cell activation but does not induce cell proliferation or cytokine release. The second signal, i.e., the co-stimulatory signal, determines T cells undergo activation/proliferation, unresponsiveness, or apoptosis. The B7-CD28 co-stimulatory signaling pathway plays an important role in regulation of T cell activation and suppression, wherein the CD28 molecules are expressed on T cells while the B7 molecules are generally present on activated antigen-presenting cells. Till now more than 10 B7 proteins have been discovered and are divided into 3 groups according to the signals and functions they transduce during T cell activation, i.e., i) co-stimulatory, ii) co-inhibitory, and iii) co-stimulatory/inhibitory.

B7-H3, also known as CD276, is a co-stimulatory/inhibitory molecule of the B7 family. When initially discovered, it was deemed as a co-stimulatory molecule that promotes T cell activation and IFN-γ production. However, studies in recent years suggest that the B7-H3 plays an inhibitory role in adaptive immunity, e.g., to suppress T cell activation/proliferation or release of effector cytokines (mainly IFN-γ and IL-2), to inhibit NK cell activity, and etc. (Prasad DVR. et al., (2004) J Immunol 173(4):2500-2506; Castriconi R. et al., (2004) Proc Natl Acad Sci USA 101(34):12640-12645). B7-H3's seemingly contradictory regulatory activity may be mediated by different receptors, but only a few researches have been done for B7-H3 receptors.

B7-H3 is a type I transmembrane protein, sharing 20%-27% similarity to the other B7 family members. It is encoded by the gene located on chromosome 15q24, and contains an extracellular domain, a transmembrane domain, and a short intracellular domain. The intracellular domain is quite short and is not known to have any signaling motif. In addition, the human B7-H3 protein may contain one or two pairs of extracellular domains due to exon duplication, resulting in two subtypes, i.e., 2IgB7-H3 and 4IgB7-H3. The previous one subtype contains one pair of immunoglobulin variable region (IgV)-like and immunoglobulin constant region (IgC)-like extracellular domains, while the latter one contains two pairs of identical IgV-like and IgC-like extracellular domains, and is the main subtype found in human cells.

The B7-H3 mRNAs are ubiquitously expressed in normal tissues, while the B7-H3 protein molecules are lowly expressed in only a few tissues (Flem-Karlsen K. et al., (2018) Trends Cancer 4(6):401-404). For example, weak staining was found in the cytoplasm of salivary gland acinar cells, gastric epithelial cells, and adrenal gland cells, and also weak staining was found in the basement membrane of stomach, gallbladder, prostate, cervix and endometrium. The B7-H3 proteins are highly expressed by several malignant tumors. Various studies have shown that the B7-H3 molecules are expressed in 33.0% of the renal cell carcinoma patients and 91.8% of hepatocellular carcinoma patients. It should be noted that the percent of patients with B7-H3 expression is quite low in renal cell carcinoma and systemic hematologic cancers (33.0% and 36.7% respectively, p<0.001) while at least 52.3% of patients of other solid cancers have B7-H3 expression. Among the gastrointestinal cancers, the percent of patients with B7-H3 expression is the highest in hepatocellular carcinoma (91.8%), and the lowest in gastric cancer (58.0%). Among the malignant urogenital cancers, the percent of patients with B7-H3 expression is the lowest in renal cell carcinoma and the highest in ovarian cancer (88.3%). Among all the cancers, 59.5% of the patients (15877/26703) are B7-H3 positive. On one hand, it was found that the pancreatic cancer patients with high B7-H3 expression have better prognosis after surgeries than those with low B7-H3 expression (Loos M. et al., (2009) BMC Cancer 9: 463). On the other hand, it is believed B7-H3 contributes to immunosuppressive tumor microenvironment, promoting cancer stem cells in e.g., squamous cell carcinoma evade from immune surveillance (Wang C. et al., (2021) Cell Stem Cell 28(9): 1597-1613; Jin MZ, Jin WL., (2020) Signal Transduct Target Ther 5(1): 166). B7-H3 also promotes cancer occurrence and development by several non-immune pathways. For example, it induces VEGFA expression in colorectal cancer cells, promotes epithelial-to-mesenchymal transition in colorectal cancer, and fuels the tumors by triggering aerobic glycolysis (Wang RQ et al., (2020) Cell Death Dis (2020) 11(1):55; Jiang B. et al., (2016) Oncotarget 7(22): 31755-31771; Lim S. et al., (2016) Cancer Res 76(8): 2231-2242).

The *in vivo* expression pattern of B7-H3 makes it a promising target for cancer treatment. Especially, high B7-H3 expression was also found in stromal cells (fibroblasts) in tumor microenvironment and tumor associated vasculature (TAV), so that the B7-H3 targeted treatment may even eliminate cancer cells with low B7-H3 expression.

Currently B7-H3 targeting therapeutics in various forms are in the clinical trial phase. Enoblituzumab is the first monoclonal antibody targeting the B7-H3 protein with optimized Fc region, which exerts the anti-tumor activity through both the antibody-dependent cell mediated cytotoxicity and T cell immune function enhancement. According to the data published in 2018 annual report of Society for Immunotherapy of Cancer (SITC), the objective response rate (ORR) of the combination therapy of enoblituzumab and an anti-PD-L1 antibody was 33.3% in patients of squamous cell carcinoma of head and neck (SCCHN) in the clinical trial, and the ORR achieved 35.7% in patients of non-small cell lung cancer (NSCLC) with low PD-L1 expression (< 1%). Especially, the patients that received prior anti-PD-1/L1 antibody treatment became responsive to such therapy (partial remission, PR), and the same percent of patients had the disease under control as compared to those that were subject to the anti-PD-1/L1 treatment for the first time. ¹³¹I labeled monoclonal antibody 8H9 (Omburtamab, Y-mAbs) has been used to treat patients of metastatic central nervous system neuroblastoma (NCT00089245) and those of desmoplastic small round cell tumor (NCT01099644) via intraperitoneal injection, and was well tolerated. Similarly, ¹²⁴I labeled monoclonal antibody 8H9 was tested in diffuse intrinsic pontine glioma (NCT01502917), and was also well tolerated. Some other B7-H3 targeting therapeutics, including the small molecular drugs, mono-specific antibodies, bi-specific antibodies, antibody-drug conjugates, CAR-T and the like, are being tested in clinical trials of solid tumors such as melanoma, prostate cancer, head and neck squamous cell carcinoma, non-small cell lung cancer, central nervous system cell tumors, glioblastoma, desmoplastic small round cell tumor, medulloblastoma and the like (Zhou WT, Jin WL. (2021) Front Immunol 12: 701006).

However, despite of the B7-H3 targeting therapies' broad-spectrum anti-tumor effects, these therapies are still in the early-stage clinical trials, and clinically efficacious to only a small part of the patients. Furthermore, it should be noted that, these therapies may show very good therapeutic effects in some patients at the beginning, and resistance may occur later. To find out the causes of the non-responsiveness and resistance development in certain patients, and then to develop new treatment means and schemes, are critical for further cancer treatment. There is still a huge demand in the cancer treatment field for more anti-B7-H3 antibodies with different properties and efficacy characteristics, such as anti-B7-H3 antibodies with different binding affinity, blocking activity, and even different binding epitopes.

Citation or identification of any document in this application is not an admission that such document is available as prior art to the present invention.

### SUMMARY OF THE INVENTION

The present application provides an isolated monoclonal antibody, such as a human or chimeric monoclonal antibody, that binds to B7-H3 (e.g., human B7-H3, monkey B7-H3 or mouse B7-H3), which, as compared to the prior art antibodies such as enoblituzumab, has comparable or higher human/monkey B7-H3 binding affinity/binding activity, comparable or higher B7-H3⁺ cell binding activity, comparable or higher immune cell (e.g., NK cell) activation activity, comparable or higher ability to induce antibody dependent cell mediated cytotoxicity (ADCC), comparable or higher *in vivo* anti-tumor effect. The monoclonal antibody of the present application may be internalized by B7-H3⁺ cells, and its binding specificity to human B7-H3 is superior to that of the prior art antibodies such as enoblituzumab.

The antibody of the present application may have several applications, including treating B7-H3 associated diseases such as cancers.

Therefore, in one aspect, the disclosure provides an isolated monoclonal antibody (e.g., a human antibody) or an antigen binding portion thereof, that binds to B7-H3), which may comprise i) a heavy chain variable region, wherein the heavy chain variable region comprises a VH CDR1 region, a VH CDR2 region and a VH CDR3 region, wherein the VH CDR1 region, the VH CDR2 region and the VH CDR3 region may comprise the amino acid sequences of (1) SEQ ID NOs: 1, 2 and 3, respectively; (2) SEQ ID NOs: 1, 7 and 8, respectively; (3) SEQ ID NOs: 12, 13 and 14, respectively; (4) SEQ ID NOs: 18, 19 and 20, respectively; or (5) SEQ ID NOs: 23, 24 and 25, respectively, or amino acid sequences having at least 80%, 85%, 90%, 91%, 92%, 93%, 94%, 95%, 96%, 97%, 98%, or 99% identity to the above sequences; and/or ii) a light chain variable region, wherein the light chain variable region comprises a VL CDR1 region, a VL CDR2 region and a VL CDR3 region, wherein the VL CDR1 region, the VL CDR2 region, and the VL CDR3 region may comprise the amino acid sequences of (1) SEQ ID NOs: 4, 5 and 6, respectively; (2) SEQ ID NOs: 9, 10 and 11, respectively; (3) SEQ ID NOs: 15, 16 and 17, respectively; (4) SEQ ID NOs: 9, 21 and 22, respectively; or (5) SEQ ID NOs: 26, 27 and 28, respectively, or amino acid sequences having at least 80%, 85%, 90%, 91%, 92%, 93%, 94%, 95%, 96%, 97%, 98%, or 99% identity to the above sequences.

The isolated monoclonal antibody or antigen binding portion thereof the present application may comprise a heavy chain variable region and a light chain variable region, wherein the VH CDR1 region, the VH CDR2 region, the VH CDR3 region, the VL CDR1 region, the VL CDR2 region, and the VL CDR3 region may comprise the amino acid sequences of (1) SEQ ID NOs: 1, 2, 3, 4, 5 and 6, respectively; (2) SEQ ID NOs: 1, 7, 8, 9, 10 and 11, respectively; (3) SEQ ID NOs: 12, 13, 14, 15, 16 and 17, respectively; (4) SEQ ID NOs: 18, 19, 20, 9, 21 and 22, respectively; or (5) SEQ ID NOs: 23, 24, 25, 26, 27 and 28, respectively, or amino acid sequences having at least 80%, 85%, 90%, 91%, 92%, 93%, 94%, 95%, 96%, 97%, 98%, or 99% identity to the above sequences.

The heavy chain variable region of the isolated monoclonal antibody or antigen binding portion thereof of the disclosure may comprise an amino acid sequence having at least 80%, 85%, 90%, 91%, 92%, 93%, 94%, 95%, 96%, 97%, 98%, 99% or 100% identity to SEQ ID NOs: 29, 31, 33, 35 or 37.

The light chain variable region of the isolated monoclonal antibody or antigen binding portion thereof of the disclosure may comprise an amino acid sequence having at least 80%, 85%, 90%, 91%, 92%, 93%, 94%, 95%, 96%, 97%, 98%, 99% or 100% identity to SEQ ID NOs: 30, 32, 34, 36 or 38.

The heavy chain variable region and the light chain variable region of the isolated monoclonal antibody or antigen binding portion thereof of the present application may comprise the amino acid sequences of (1) SEQ ID NOs: 29 and 30, respectively; (2) SEQ ID NOs: 31 and 32, respectively; (3) SEQ ID NOs: 33 and 34, respectively; (4) SEQ ID NOs: 35 and 36, respectively; or (5) SEQ ID NOs: 37 and 38, respectively, or amino acid sequences having at least 80%, 85%, 90%, 91%, 92%, 93%, 94%, 95%, 96%, 97%, 98%, or 99% identity to the above sequences.

In another aspect, the disclosure provides an isolated monoclonal antibody (e.g., a human antibody) or an antigen binding portion thereof, that binds to B7-H3, which comprises i) a heavy chain variable region, wherein the heavy chain variable region comprises a VH CDR1 region, a VH CDR2 region and a VH CDR3 region, wherein the VH CDR1 region, the VH CDR2 region and the VH CDR3 region have identity to a VH CDR1 region, a VH CDR2 region and a VH CDR3 region of a specified VH sequence, and/or ii) a light chain variable region, wherein the light chain variable region comprises a VL CDR1 region, a VL CDR2 region and a VL CDR3 region, wherein the VL CDR1 region, the VL CDR2 region, and the VL CDR3 region have identity to a VL CDR1 region, a VL CDR2 region, and a VL CDR3 region of a specified VL sequence; wherein the specified VH sequence and the specified VL sequence are one of the following: (1) the specified VH sequence and the specified VL sequence set forth in SEQ ID NOs: 29 and 30, respectively; (2) the specified VH sequence and the specified VL sequence set forth in SEQ ID NOs: 31 and 32, respectively; (3) the specified VH sequence and the specified VL sequence set forth in SEQ ID NOs: 33 and 34, respectively; (4) the specified VH sequence and the specified VL sequence set forth in SEQ ID NOs: 35 and 36, respectively; (5) the specified VH sequence and the specified VL sequence set forth in SEQ ID NOs: 37 and 38, respectively.

In one embodiment, the isolated monoclonal antibody or antigen binding portion thereof of the disclosure may comprise a heavy chain constant region and/or a light chain constant region. The heavy chain constant region may be an IgG1, IgG2, IgG3 or IgG4 heavy chain constant region, or a functional fragment thereof. Further, the heavy chain constant region may be engineered to e.g., have enhanced FcR binding activity, to enhance the ADCC against B7-H3⁺ cells. For example, the heavy chain constant region may be introduced with SI mutation (S239D and I332E mutations, according to EU numbering) or VLPLL mutation (L235V, F243L, R292P, Y300L and P396L, according to EU numbering). In one embodiment, the heavy chain constant region may be a wildtype human IgG1 constant region containing the amino acid sequence of SEQ ID NO: 43. In one embodiment, the heavy chain constant region may be a human IgG1 constant region containing the amino acid sequence of SEQ ID NO: 44 (L235V, F243L, R292P, Y300L and P396L, according to EU numbering). The light chain constant region may be a κ constant region, e.g., a human κ constant region containing the amino acid sequence of SEQ ID NO: 45, or a functional fragment thereof. The N terminus of the heavy chain constant region is linked to the C terminus of the heavy chain variable region, and the N terminus of the light chain constant region is linked to the C terminus of the light chain variable region.

The antibody or the antigen binding portion thereof of the present application may be recombinantly expressed in e.g., certain mammalian cell lines, for afucosylation. The cell lines for expressing afucosylated antibody or antigen binding portion thereof include, but not limited to, the Slc35C1 knock-out cell line, the FUT8 knock-out cell line, the Lec13 cell line (variant CHO cell line), the rat myeloma cell line YB2/0, the cell line containing small interfering RNAs specifically against gene FUT8, and the cell line co-expressing β-1,4-N-acetylglucosaminyltransferase III and Golgi α-mannosidase II.

The antibody of the present disclosure, in certain embodiments, may comprise two heavy chains and two light chains, or consist of two heavy chains and two light chains, wherein each heavy chain comprises the heavy chain constant region, heavy chain variable region and/or CDR sequences mentioned above, and each light chain comprises the light chain constant region, light chain variable region and/or CDR sequences mentioned above. In certain embodiments, the antibody of the present disclosure may be a single chain variable fragment, or consists of antibody fragments, such as a Fab or F(ab')₂ fragment.

The present application further provides an immunoconjugate that comprises the antibody or antigen binding portion thereof of the disclosure, wherein the antibody or antigen binding portion thereof is linked to a therapeutic agent such as a cytotoxin or an anti-cancer agent. The disclosure further provides a bispecific molecule that comprises the antibody or antigen binding portion thereof of the disclosure, wherein the antibody or antigen binding portion thereof is linked to a second functional moiety (e.g., a second antibody), wherein the second functional moiety has a different binding specificity than the antibody or antigen binding portion thereof of the disclosure. In another respect, the antibody or antigen binding portion thereof of the disclosure may be part of a chimeric antigen receptor (CAR) or an engineered T cell receptor (TCR). Also provided is an immune cell that comprises the CAR and/or the TCR above, including T cells, NK cells or the like. The antibody or antigen binding portion thereof of the disclosure may also be encoded by an oncolytic virus or carried by an oncolytic virus.

The present application further encompasses a nucleic acid molecule encoding the antibody or antigen binding portion thereof, or the bispecific molecule of the disclosure, as well as an expression vector containing the nucleic acid and a host cell containing the expression vector.

In one embodiment, the expression vector of the disclosure contains one or more nucleic acids mentioned above. In another embodiment, the expression vector of the disclosure contains two nucleic acids mentioned above, which encode the VH and the VL, respectively, that can jointly bind to the B7-H3. In another embodiment, the disclosure provides a pair of vectors, wherein each vector contains one of the nucleic acids mentioned above, the pair of vectors together encode the VH and the VL that can bind to B7-H3.

The disclosure furthermore provides a method for preparing the antibody or the antigen binding portion thereof, or the bispecific molecule of the disclosure, using the host cell containing the expression vector above, which comprises (i) expressing the antibody, the antigen binding portion thereof or the bispecific molecule in the host cell, and (ii) isolating the antibody, the antigen binding portion thereof or the bispecific molecule from the host cell or its culture.

The present application further provides a pharmaceutical composition, comprising the antibody or antigen binding portion thereof, the immunoconjugate, the bispecific molecule, the immune cell, the oncolytic virus, the nucleic acid molecule, the expression vector, or the host cell of the disclosure, and a pharmaceutically acceptable carrier.

In one aspect, the present application provides a method for treating or alleviating a B7-H3 associated disease in a subject, comprising administering to the subject a therapeutically effective amount of the pharmaceutical composition of the disclosure. The B7-H3 associated disease may be a cancer or an autoimmune disease. The cancer may be a solid tumor, including, but not limited to, breast cancer, melanoma, prostate cancer, head and neck squamous cell carcinoma, lung cancer, non-small cell lung cancer, central nervous system neuroblastoma, glioblastoma, desmoplastic small round cell tumor, diffuse pontine glioma, medulloblastoma, pancreatic cancer, liver cancer, colorectal cancer, non-Hodgkin lymphoma, esophageal cancer, ovarian cancer, bladder cancer, small cell carcinoma, endometrial cancer, kidney cancer, gastric cancer, acute myeloid leukemia, hepatocellular carcinoma, hypopharyngeal squamous cell carcinoma, urothelial cell carcinoma. The autoimmune disease includes, but not limited to, asthma. In certain embodiments, the antibody or antigen binding portion thereof of the disclosure may be administered with at least one additional anti-cancer antibody, such as an anti-PD-1 antibody, an anti-PD-L1 antibody, an anti-4-1BB antibody and the like. In another embodiment, the antibody or antigen binding portion thereof of the disclosure may be administered with a cytokine (e.g., IL-2 and or IL-21) or a co-stimulatory antibody (e.g., an anti-CD137 antibody and/or an anti-GITR antibody). In another embodiment, the antibody or the antigen binding portion thereof of the disclosure may be administered with a chemotherapeutic agent, which may be a cytotoxic agent. The antibody of the disclosure may be e.g., a human antibody.

In one aspect, the present disclosure provides a method of enhancing an immune response in a subject, comprising administering to the subject in need thereof an effective amount of the antibody or the antigen binding portion thereof of the disclosure. The enhancement of an immune response includes activation of immune cells, including NK cells and the like.

All documents cited or referenced in the present application (including without limitation all literature documents, patents, published patent applications cited herein) ("herein cited documents"), all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference. Any Genbank sequences mentioned in this disclosure are incorporated by reference.

It should be noted that in this disclosure and particularly in the claims, terms such as "comprise", "contain" and the like can have the meaning attributed to it in Chinese Patent law; and the terms such as "consist essentially of" have the meaning ascribed to them in Chinese Patent law, e.g., they allow for elements not explicitly recited, but exclude elements that are found in the prior art or that affect a basic or novel characteristic of the invention.

Other features and advantages of the instant disclosure will be apparent from the following detailed description and examples, which should not be construed as limiting. The contents of all references, Genbank entries, patents and published patent applications cited throughout this application are expressly incorporated herein by reference.

### BRIEF DESCRIPTION OF THE DRAWINGS

The following detailed description, given by way of example, but not intended to limit the invention solely to the specific embodiments described, may best be understood in conjunction with the accompanying drawings.
FIG. 1 shows the binding activity of fully human anti-B7-H3 antibodies to B7-H3⁺ tumor cells, including A549 cells (A), NCI-H520 cells (B), HepG2 cells (C), SK-OV-3 cells (D) and MCF-7 cells (E).
FIG. 2 shows the binding activity of fully human anti-B7-H3 antibodies to B7-H3⁻ tumor cells, including Jurkat cells (A), Daudi cells (B) and Raji cells (C).
FIG. 3 shows the binding activity of fully human anti-B7-H3 antibodies to B7-H3⁺ normal cells, including human prostate smooth muscle cells (A), human dermal fibroblasts (B), human aorta smooth muscle cells (C) and human dendritic cells (D).
FIG. 4 shows the binding activity of fully human anti-B7-H3 antibodies with or without Fc engineering, namely 6B5-WT, 6B5-AF and 6B5-Mut, to HUVEC cells (A) and NCI-H520 cells (C), and the binding activity of 10D7-WT, 10D7-AF and 10D7-Mut to HUVEC cells (B) and NCI-H520 cells (D).
FIG. 5 shows the ability of fully human anti-B7-H3 antibodies with or without Fc engineering, namely 10D7-WT, 10D7-AF and 10D7-Mut to induce NCI-H520 cell killing by NK cells (A) and to activate NK cells (C), and the ability of 6B5-WT, 6B5-AF and 6B5-Mut to induce NCI-H520 cell killing by NK cells (B) and to activate NK cells (D).
FIG. 6 shows the ability of fully human anti-B7-H3 antibodies with or without Fc engineering, namely 10D7-WT, 10D7-AF and 10D7-Mut to induce NCI-H520 cell killing by PBMCs (A), and the ability of 6B5-WT, 6B5-AF and 6B5-Mut to induce NCI-H520 cell killing by PBMCs (B).
FIG. 7 shows the ability of afucosylated fully human anti-B7-H3 antibodies, 10D7-AF and 6B5-AF, to induce HUVEC cell killing by NK cell (A) or PBMCs (B), and the release of IFN-γ by PBMCs during HUVEC cell killing (C).
FIG. 8 shows the ability of afucosylated fully human anti-B7-H3 antibodies, 10D7-AF and 6B5-AF, to induce killing of mature dendritic cell by NK cells.
FIG. 9 shows the internalization of fully human anti-B7-H3 antibodies, by NCI-H520 cells (A) and A549 cells (B).
FIG. 10 is an average linkage clustering-derived tree diagram of the B7-H3 epitopes bound by the fully human anti-B7-H3 antibodies.
FIG. 11 shows the binding affinity of the 6B5 antibodies to B7 family members by SPR.
FIG. 12 shows the tumor size change in mice with human B7-H3 gene that were treated by the fully human anti-B7-H3 antibodies 10F5-Mut (A), 6B5-Mut, 15F11-Mut (B).
FIG. 13 shows the representative tissue staining results of the afucosylated fully human anti-B7-H3 antibody 6B5-AF.

### DETAILED DESCRIPTION OF THE INVENTION

To ensure that the present disclosure may be more readily understood, certain terms are first defined. Additional definitions are set forth throughout the detailed description.

The term "B7-H3" refers to B7 homolog 3, also known as Cluster of Differentiation 276 (CD276), which can be divided into two subtypes, i.e., 4Ig and 2Ig, according to the extracellular domain structure. The term comprises variants, homologs, orthologs and paralogs. For example, an antibody specific for human B7-H3 may, in certain cases, cross-react with a B7-H3 protein from another species such as monkey. In other embodiments, an antibody specific for the human B7-H3 protein may be completely specific for the human B7-H3 protein and exhibit no cross-reactivity to other species or of other types, or may cross-react with the B7-H3 protein from certain other species but not all other species.

The term "human B7-H3" refers to a B7-H3 protein having an amino acid sequence from the human, such as the B7-H3 (4Ig) protein containing the amino acid sequence having NCBI accession no. NP_001019907.1 (Kanayama T. et al., (2021) Sci Rep 11(1): 18802) or the B7-H3 (2Ig) protein containing the amino acid sequence having NCBI accession no. NP_001316557.1 (Fu M. et al., (2021) J Transl Med 19(1):404). The term "monkey B7-H3" refers to a B7-H3 protein having an amino acid sequence from the monkey, such as the B7-H3 containing the amino acid sequence having NCBI accession no. XP_015308534.1. The term "mouse B7-H3" refers to a B7-H3 protein having an amino acid sequence from the mouse, such as the B7-H3 protein containing the amino acid sequence having NCBI accession no. NP_598744.1 (Cheng N. et al., (2021) Biochem Pharmacol 183: 114298).

The term "antibody" as used herein is intended to include the full-length antibodies of IgG, IgA, IgD, IgE and IgM, and any of the antigen binding fragments thereof (i.e., the antigen binding portion). A full-length antibody is a glycoprotein which may at least comprise two heavy (H) chains and two light (L) chains inter-connected by disulfide bonds. Each heavy chain may be comprised of a heavy chain variable region (abbreviated herein as V_{H}) and a heavy chain constant region. The heavy chain constant region may be comprised of three domains, C_{H1}, C_{H2} and C_{H3}. Each light chain may be comprised of a light chain variable region (abbreviated herein as V_{L}) and a light chain constant region. The light chain constant region may be comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant region of the antibody may mediate the binding of the immunoglobulin to host tissues or factors, including various cells of the immune system (e.g., effector cells) and the first component (C1q) of the classical complement system. The "functional fragment" of an antibody constant region refers to a fragment of the constant region that retains certain required function(s), such as the fragment of the heavy chain constant region that retains the FcR/complement system component binding activity.

The term "antigen binding portion" of an antibody (or simply "antibody portion"), as used herein, refers to one or more fragments of an antibody that retain the ability to specifically bind to an antigen (e.g., the B7-H3 protein). It has been shown that the antigen binding function of an antibody can be performed by fragments of a full-length antibody. Examples of binding fragments encompassed within the term "antigen binding portion" of an antibody include (i) a Fab fragment, a monovalent fragment consisting of the V_{L}, V_{H}, C_{L} and C _{H1} domains; (ii) a F(ab')₂ fragment, a bivalent fragment which may comprise two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546); (vi) an isolated complementarity determining region (CDR); and (viii) a nanobody, a heavy chain variable region containing a single variable domain and two constant domains. Furthermore, although the two domains of the Fv fragment, V_{L} and V_{H}, are coded by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al., (1988) Science 242:423-426; and Huston et al., (1988) Proc. Natl. Acad. Sci. USA 85:5879-5883). Such single chain antibodies are also intended to be encompassed within the term "antigen binding portion" of an antibody. These antibody fragments are obtained using conventional techniques known to those with skill in the art, and the fragments are screened for utility in the same manner as are intact antibodies.

An "isolated antibody", as used herein, is intended to refer to an antibody that is substantially free of other antibodies having different antigenic specificities. For example, an isolated antibody that specifically binds a B7-H3 protein is substantially free of antibodies that specifically bind antigens other than B7-H3 proteins. An isolated antibody that specifically binds a human B7-H3 protein may, however, have cross-reactivity to other antigens, such as B7-H3 proteins from other species. Moreover, an isolated antibody can be substantially free of other cellular material and/or chemicals.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from human germline heavy chain only antibody sequences. Furthermore, if the antibody contains a constant region, the constant region also is derived from human germline antibody sequences. The human antibodies of the disclosure can include amino acid residues not encoded by human germline antibody sequences (e.g., mutations introduced by random or site-specific mutagenesis *in vitro* or by somatic mutation *in vivo*). However, the term "human antibody", as used herein, is not intended to include antibodies in which CDR sequences derived from the germline of another mammalian species have been grafted onto human framework sequences.

The term "chimeric antibody" refers to an antibody made by combining genetic material from a nonhuman source with genetic material from a human being. Or more generally, a chimeric antibody is an antibody having genetic material from a certain species with genetic material from another species.

The phrases "an antibody recognizing an antigen" and "an antibody specific for an antigen" are used interchangeably herein with the term "an antibody which binds specifically to an antigen."

As used herein, an antibody that "specifically binds to human B7-H3" is intended to refer to an antibody that binds to human B7-H3 protein (and the B7-H3 from another non-human species) but does not substantially bind to non-B7-H3 proteins. Preferably, the antibody binds to the human B7-H3 protein with "high affinity", namely with a K_{D} of 5.0 ×10⁻⁸ M or less.

The term "does not substantially bind" to a protein or a cell, means does not bind or does not bind with a high affinity to the protein or cell, i.e. binds to the protein or cell with a K_{D} of 1.0 × 10⁻⁶ M or more, more preferably 1.0 × 10⁻⁵ M or more, more preferably 1.0 × 10⁻⁴ M or more, 1.0 × 10⁻³ M or more, more preferably 1.0 × 10⁻² M or more.

The term "EC₅₀", also known as half maximal effective concentration, refers to the concentration of an antibody which gives half (50%)-maximal response.

The term "IC₅₀", refers to the half-maximal inhibitory concentration, which is the concentration of an agent or an inhibitory agent that inhibit a specific biological process by 50%.

The term "antibody dependent cytotoxicity", "antibody dependent cell mediated cytotoxicity", or "ADCC" refers to a cell mediated immune defense that the effector immune cells actively lyse the target cells whose cell surface antigens are bound by the antibodies such as the anti-B7-H3 antibodies of the disclosure.

The term "subject" includes any human or nonhuman animal. The term "nonhuman animal" includes all vertebrates, e.g., mammals and non-mammals, such as non-human primates, sheep, dogs, cats, cows, horses, chickens, amphibians, and reptiles, although mammals are preferred, such as non-human primates, sheep, dogs, cats, cows and horses.

The term "therapeutically effective amount" refers to the amount of an antibody of the disclosure that is sufficient to prevent or alleviate the symptoms associated with a disease or disorder (e.g., cancer). The therapeutically effective amount is related to a disease to be treated, and those skilled in the art can readily identify the actual effective amount.

Various aspects of the disclosure are described below in further detail.

The antibody or antigen binding portion thereof of the disclosure is able to bind to the B7-H3 of human, monkey and the like, including the B7-H3⁺ cells, and the binding activity/binding affinity is comparable to or better than that of the prior art antibodies such as enoblituzumab. In addition, compared to the prior art antibodies such as enoblituzumab, the antibody or antigen binding portion thereof of the disclosure has comparable or higher immune cell (e.g., NK cell) activation ability, comparable or higher antibody dependent cell mediated cytotoxicity (ADCC) induction ability, comparable or better *in vivo* anti-tumor effect. The monoclonal antibody or antigen binding portion thereof of the disclosure may be internalized by B7-H3⁺ cells, and its binding specificity to human B7-H3 is better than the prior art antibodies such as enoblituzumab.

The preferable antibody of the disclosure is a monoclonal antibody. The antibody may be e.g., a human or chimeric monoclonal antibody.

The exemplary antibody or the antigen binding portion thereof of the disclosure is structurally and chemically characterized below.\

The heavy chain variable region CDRs and the light chain variable region CDRs of the antibody or antigen binding portion thereof of the disclosure have been defined by the Kabat numbering system. The SEQ ID NO of the CDR amino acid sequences, and the SEQ ID NO of the heavy/light chain amino acid sequences are set forth in Table 1A. The heavy chain variable region CDRs and the light chain variable region CDRs of the antibody or the antigen binding portion thereof of the disclosure can also be determined by the Chothia numbering system, and the SEQ ID NO of the CDR amino acid sequences as determined by such system are set forth in Table 1B.

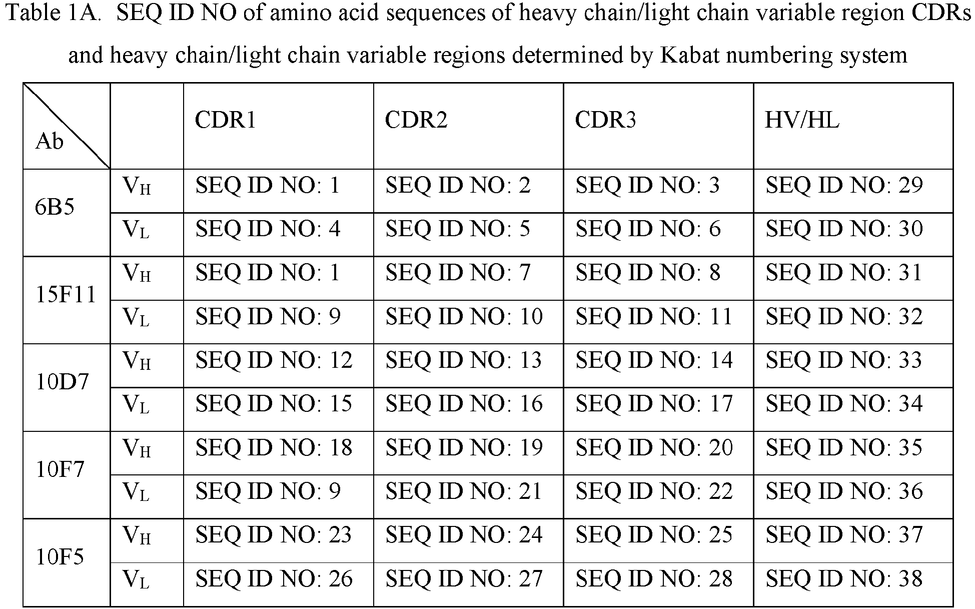

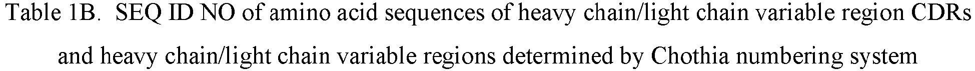

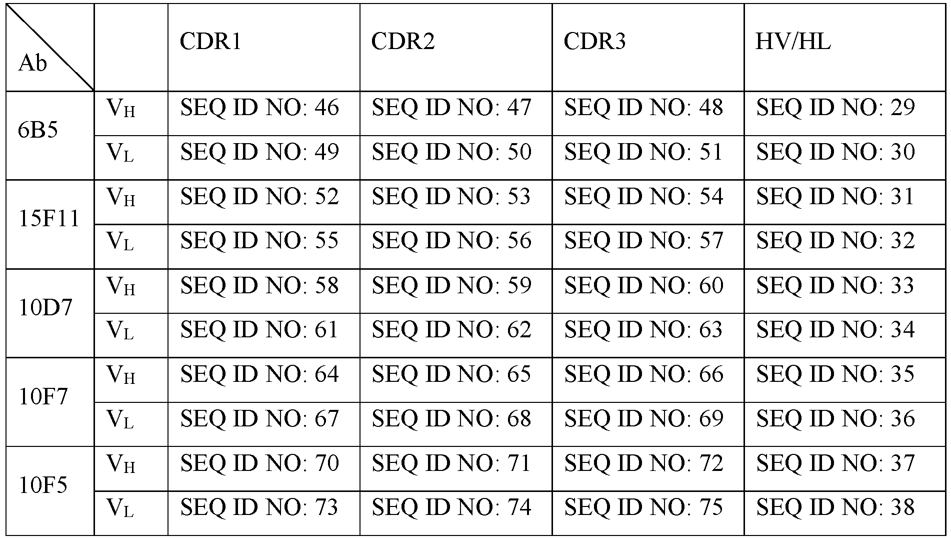

In addition to the exemplary CDRs above determined by the Kabat and Chothia numbering systems, the heavy chain/light chain variable regions of the antibody of the disclosure may contain CDR regions determined by other numbering systems based on SEQ ID NOs: 29, 30, 31, 32, 33, 34, 35, 36, 37 and 38. It is well known in the art that the heavy chain variable region and light chain variable region CDRs may also be determined by e.g., IMGT, AbM or Contact numbering system/method.

The antibody of the disclosure may contain a heavy chain constant region, such as an IgG1 constant region, e.g., a human IgG1 constant region containing e.g., the amino acid sequence of SEQ ID NO: 43. The constant region may be engineered to have enhanced FcR binding ability, such as human IgG1 constant region containing the amino acid sequence of SEQ ID NO: 44 (L235V, F243L, R292P, Y300L and P396L, according to EU numbering). The light chain constant region may be a κ constant region, such as a human κ constant region which may contain the amino acid sequence of SEQ ID NO: 45.

The V_{H} and/or V_{L} sequences (or CDR sequences) of other anti-B7-H3 antibodies which bind to human B7-H3 can be "mixed and matched" with the V_{H} and V_{L} sequences (or CDR sequences) of the anti-B7-H3 antibody of the present disclosure. Preferably, when V_{H} and V_{L} chains (or the CDRs within such chains) are mixed and matched, a V_{H} sequence from a particular V_{H}/V_{L} pairing is replaced with a structurally similar V_{H} sequence. Likewise, preferably a V_{L} sequence from a particular V_{H}/V_{L} pairing is replaced with a structurally similar V_{L} sequence.

Accordingly, in one embodiment, an antibody of the disclosure, or an antigen binding portion thereof, comprises:
(a) a heavy chain variable region comprising an amino acid sequence set forth in Table 1; and
(b) a light chain variable region comprising an amino acid sequence set forth in Table 1, or the V_{L} of another anti-B7-H3 antibody, wherein the antibody specifically binds human B7-H3.

In another embodiment, an antibody of the disclosure, or an antigen binding portion thereof, comprises:
(a) the CDR1, CDR2, and CDR3 of the heavy chain variable region set forth in Table 1; and
(b) the CDR1, CDR2, and CDR3 of the light chain variable region set forth in Table 1 or the CDRs of another anti-B7-H3 antibody, wherein the antibody specifically binds human B7-H3.

In yet another embodiment, the antibody or antigen binding portion thereof of the disclosure comprises the heavy chain variable CDR2 region of the anti-B7-H3 antibody combined with CDRs of other antibodies which bind human B7-H3, e.g., CDR1 and/or CDR3 from the heavy chain variable region, and/or CDR1, CDR2, and/or CDR3 from the light chain variable region of another anti-B7-H3 antibody.

In addition, it is well known in the art that the CDR3 domain, independently from the CDR1 and/or CDR2 domain(s), alone can determine the binding specificity of an antibody for a cognate antigen and that multiple antibodies can predictably be generated having the same binding specificity based on a common CDR3 sequence. See, e.g., Klimka et al., British J. of Cancer 83(2):252-260 (2000); Beiboer et al., J. Mol. Biol. 296:833-849 (2000); Rader et al., Proc. Natl. Acad. Sci. U.S.A. 95:8910-8915 (1998); Barbas et al., J. Am. Chem. Soc. 116:2161-2162 (1994); Barbas et al., Proc. Natl. Acad. Sci. U.S.A. 92:2529-2533 (1995); Ditzel et al., J. Immunol. 157:739-749 (1996); Berezov et al., BIAjournal 8: Scientific Review 8 (2001); Igarashi et al., J. Biochem (Tokyo) 117:452-7 (1995); Bourgeois et al., J. Virol 72:807-10 (1998); Levi et al., Proc. Natl. Acad. Sci. U.S.A. 90:4374-8 (1993); Polymenis and Stoller, J. Immunol. 152:5218-5329 (1994) and Xu and Davis, Immunity 13:37-45 (2000). See also, U.S. Pat. Nos. 6,951,646; 6,914,128; 6,090,382; 6,818,216; 6,156,313; 6,827,925; 5,833,943; 5,762,905 and 5,760,185. These references were hereby incorporated by reference in their entirety.

In another embodiment, the antibody of the disclosure may comprise the CDR2 of the heavy chain variable region of the anti-B7-H3 antibody and at least the CDR3 of the heavy and/or light chain variable region of the anti-B7-H3 antibody, or the CDR3 of the heavy and/or light chain variable region of another anti-B7-H3 antibody, wherein the antibody is capable of specifically binding to human B7-H3. These antibodies preferably (a) compete for binding with B7-H3; (b) retain the functional characteristics; (c) bind to the same epitope; and/or (d) have a similar binding affinity as the anti-B7-H3 antibody of the present disclosure. In yet another embodiment, the antibodies further may comprise the CDR2 of the light chain variable region of the anti-B7-H3 antibody, or the CDR2 of the light chain variable region of another anti-B7-H3 antibody, wherein the antibody is capable of specifically binding to human B7-H3. In another embodiment, the antibodies of the disclosure may include the CDR1 of the heavy and/or light chain variable region of the anti-B7-H3 antibody, or the CDR1 of the heavy and/or light chain variable region of another anti-B7-H3 antibody, wherein the antibody is capable of specifically binding to human B7-H3.

In another embodiment, an antibody of the disclosure may comprise a heavy and/or light chain variable region sequences of CDR1, CDR2 and CDR3 sequences which differ from those of the anti-B7-H3 antibodies of the present disclosure by one or more conservative modifications. It is understood in the art that certain conservative sequence modification can be made which do not remove antigen binding. See, e.g., Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem.32:6862-35*;* Adib-Conquy et al., (1998) Int. Immunol. 10:341-6 and Beers et al., (2000) Clin. Can. Res. 6:2835-43.

Accordingly, in one embodiment, the antibody comprises a heavy chain variable region and/or a light chain variable region, wherein the heavy chain variable region and the light chain variable region each contain CDR1, CDR2, and CDR3, wherein:
(a) the heavy chain variable region CDR1 comprises a sequence listed in Table 1 above, and/or conservative modifications thereof; and/or
(b) the heavy chain variable region CDR2 comprises a sequence listed in Table 1 above, and/or conservative modifications thereof; and/or
(c) the heavy chain variable region CDR3 comprises a sequence listed in Table 1 above, and conservative modifications thereof; and/or
(d) the light chain variable region CDR1, and/or CDR2, and/or CDR3 comprise the sequence(s) listed in Table 1 above; and/or conservative modifications thereof; and
(e) the antibody specifically binds human B7-H3.

The antibody of the disclosure may contain one or more of the following functional characteristics, such as the high affinity and high binding specificity to human B7-H3, and enhanced FcR binding affinity and thus enhanced ability to induced ADCC against B7-H3⁺ tumor cells.

In certain embodiments, the antibody or the antigen binding portion thereof may be e.g., human or chimeric.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody of the disclosure can be replaced with other amino acid residues from the same side chain family and the altered antibody can be tested for retained function (i.e., the functions set forth above) using the functional assays described herein.

Antibodies of the disclosure can be prepared using an antibody having one or more of the V_{H}/V_{L} sequences of the anti-B7-H3 antibody of the present disclosure as starting material to engineer a modified antibody. An antibody can be engineered by modifying one or more residues within one or both variable regions (i.e., V_{H} and/or V_{L}), for example within one or more CDR regions and/or within one or more framework regions. Additionally or alternatively, an antibody can be engineered by modifying residues within the constant region(s), for example to alter the effector function(s) of the antibody.

In certain embodiments, CDR grafting can be used to engineer variable regions of antibodies. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann et al., (1998) Nature 332:323-327; Jones et al., (1986) Nature 321:522-525; Queen et al., (1989) Proc. Natl. Acad, U.S.A. 86:10029-10033; U.S. Pat. Nos. 5,225,539; 5,530,101; 5,585,089; 5,693,762 and 6,180,370).

Accordingly, another embodiment of the disclosure pertains to an isolated monoclonal antibody, or antigen binding portion thereof, comprising a heavy chain variable region comprising CDR1, CDR2, and CDR3 sequences comprising the sequences of the present disclosure, as described above, and/or a light chain variable region comprising CDR1, CDR2, and CDR3 sequences comprising the sequences of the present disclosure, as described above. While these antibodies contain the V_{H} and V_{L} CDR sequences of the monoclonal antibody of the present disclosure, they can contain different framework sequences.

Such framework sequences can be obtained from public DNA databases or published references that include germline antibody gene sequences. For example, germline DNA sequences for human heavy and light chain variable region genes can be found in the "VBase" human germline sequence database (available on the Internet at www.mrc-cpe.cam.ac.uk/vbase), as well as in Kabat *et al.,* (1991), cited supra; Tomlinson et al., (1992) J. Mol. Biol. 227:776-798; and Cox et al., (1994) Eur. J. Immunol. 24:827-836; the contents of each of which are expressly incorporated herein by reference. As another example, the germline DNA sequences for human heavy and light chain variable region genes can be found in the Genbank database. For example, the following heavy chain germline sequences found in the HCo7 HuMAb mouse are available in the accompanying Genbank Accession Nos.: 1-69 (NG--0010109, NT--024637 & BC070333), 3-33 (NG--0010109 & NT--024637) and 3-7 (NG--0010109 & NT--024637). As another example, the following heavy chain germline sequences found in the HCo12 HuMAb mouse are available in the accompanying Genbank Accession Nos.: 1-69 (NG--0010109, NT--024637 & BC070333), 5-51 (NG--0010109 & NT--024637), 4-34 (NG-0010109 & NT--024637), 3-30.3 (CAJ556644) & 3-23 (AJ406678).

Antibody protein sequences are compared against a compiled protein sequence database using one of the sequence similarity searching methods called the Gapped BLAST (Altschul *et al.,* (1997), supra), which is well known to those skilled in the art.

Preferred framework sequences for use in the antibodies of the disclosure are those that are structurally similar to the framework sequences used by antibodies of the disclosure. The V_{H} CDR1, CDR2, and CDR3 sequences can be grafted onto framework regions that have the identical sequence as that found in the germline immunoglobulin gene from which the framework sequence derives, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see e.g., U.S. Pat. No. 5,530,101; 5,585,089; 5,693,762 and 6,180,370).

Another type of variable region modification is to mutate amino acid residues within the V_{H} and/or V_{L} CDR1, CDR2 and/or CDR3 regions to thereby improve one or more binding properties (e.g., affinity) of the antibody of interest. Site-directed mutagenesis or PCR-mediated mutagenesis can be performed to introduce the mutation(s) and the effect on antibody binding, or other functional property of interest, can be evaluated in *in vitro* or *in vivo* assays as known in the art. Preferably conservative modifications (as known in the art) are introduced. The mutations can be amino acid substitutions, additions or deletions, but are preferably substitutions. Moreover, typically no more than one, two, three, four or five residues within a CDR region are altered.

Accordingly, in another embodiment, the disclosure provides isolated anti-B7-H3 monoclonal antibodies, or antigen binding portions thereof, comprising a heavy chain variable region and light chain variable region comprising: (a) a V_{H} CDR1 region comprising the sequence of the present disclosure, or an amino acid sequence having one, two, three, four or five amino acid substitutions, deletions or additions; (b) a V_{H} CDR2 region comprising the sequence of the present disclosure, or an amino acid sequence having one, two, three, four or five amino acid substitutions, deletions or additions; (c) a V_{H} CDR3 region comprising the sequence of the present disclosure, or an amino acid sequence having one, two, three, four or five amino acid substitutions, deletions or additions; (d) a V_{L} CDR1 region comprising the sequence of the present disclosure, or an amino acid sequence having one, two, three, four or five amino acid substitutions, deletions or additions; (e) a V_{L} CDR2 region comprising the sequence of the present disclosure, or an amino acid sequence having one, two, three, four or five amino acid substitutions, deletions or additions; and (f) a V_{L} CDR3 region comprising the sequence of the present disclosure, or an amino acid sequence having one, two, three, four or five amino acid substitutions, deletions or additions.

Engineered antibodies of the disclosure include those in which modifications have been made to framework residues within V_{H} and/or V_{L}, e.g., to improve the properties of the antibody. The framework modification includes mutations of one or more residues in the framework region or even one or more CDR regions, to remove the T cell epitopes, to thereby reduce the potential immunogenicity of the antibody. This approach is also referred to as "de-immunization" and is described in further detail in U.S. Patent Publication No. 20030153043.

In addition, or as an alternative to modifications made within the framework or CDR regions, antibodies of the disclosure can be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the disclosure can be chemically modified (e.g., one or more chemical moieties can be attached to the antibody) or be modified to alter its glycosylation, again to alter one or more functional properties of the antibody.

In one embodiment, the hinge region of C_{H1} is modified in such that the number of cysteine residues in the hinge region is altered, e.g., increased or decreased. This approach is described further in U.S. Pat. No. 5,677,425. The number of cysteine residues in the hinge region of C_{H1} is altered to, for example, facilitate assembly of the light and heavy chains or to increase or decrease the stability of the antibody.

In another embodiment, the Fc hinge region of an antibody is mutated to increase or decrease the biological half-life of the antibody. More specifically, one or more amino acid mutations are introduced into the C_{H2}-C_{H3} domain interface region of the Fc-hinge fragment such that the antibody has impaired Staphylococcyl protein A (SpA) binding relative to native Fc-hinge domain SpA binding. This approach is described in further detail in U.S. Pat. No. 6,165,745.

In still another embodiment, the glycosylation of an antibody is modified. For example, an aglycosylated antibody can be made (i.e., the antibody lacks glycosylation). Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. See, e.g., U.S. Pat. Nos. 5,714,350 and 6,350,861.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase or reduce the ADCC ability of antibodies. Such carbohydrate modifications can be accomplished by, for example, expressing the antibody in a host cell with altered glycosylation machinery. Cells with altered glycosylation machinery have been described in the art, including, but not limited to, the Slc35C1 knock-out cell line, the FUT8 knock-out cell line, the Lec13 cell line (variant CHO cell line), the rat myeloma cell line YB2/0, the cell line containing small interfering RNAs specifically against gene fut8, and the cell line co-expressing β-1,4-N-acetylglucosaminyltransferase III and Golgi α-mannosidase II. They can be used as host cells for expressing recombinant antibodies of the disclosure, to thereby produce an antibody with altered glycosylation. The Slc35C1 knock-out cell line may be prepared by employing e.g., the fucose-removing platform developed by MabWorks, see the CHO cell line described in the US patent No.10377833B2 with the accession number CGMCC NO. 14287.

Another modification of the antibodies herein is pegylation. An antibody can be pegylated to, for example, increase the biological (e.g., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the disclosure. See, e.g., EP 0 154 316 and EP 0 401 384.

Antibodies of the disclosure can be characterized by their various physical properties, to detect and/or differentiate different classes thereof.

For example, antibodies can contain one or more glycosylation sites in either the light or heavy chain variable region. Such glycosylation sites may result in increased immunogenicity of the antibody or an alteration of the pK of the antibody due to altered antigen binding (Marshall et al (1972) Annu Rev Biochem 41:673-702; Gala and Morrison (2004) J Immunol 172:5489-94; Wallick et al (1988) J Exp Med 168:1099-109; Spiro (2002) Glycobiology 12:43R-56R; Parekh et al (1985) Nature 316:452-7; Mimura et al., (2000) Mol Immunol 37:697-706). Glycosylation has been known to occur at motifs containing an N-X-S/T sequence. In some instances, it is preferred to have an anti-B7-H3 antibody that does not contain variable region glycosylation. This can be achieved either by selecting antibodies that do not contain the glycosylation motif in the variable region or by mutating residues within the glycosylation region.

In a preferred embodiment, the antibodies do not contain asparagine isomerism sites. The deamidation of asparagine may occur on N-G or D-G sequences and result in the creation of an isoaspartic acid residue that introduces a link into the polypeptide chain and decreases its stability (isoaspartic acid effect).

Each antibody will have a unique isoelectric point (pI), which generally falls in the pH range between 6 and 9.5. The pI for an IgG1 antibody typically falls within the pH range of 7-9.5 and the pI for an IgG4 antibody typically falls within the pH range of 6-8. There is speculation that antibodies with a pI outside the normal range may have some unfolding and instability under *in vivo* conditions. Thus, it is preferred to have an anti-B7-H3 antibody that contains a pI value that falls in the normal range. This can be achieved either by selecting antibodies with a pI in the normal range or by mutating charged surface residues.

In another aspect, the disclosure provides a nucleic acid molecule that encodes the heavy and/or light chain variable regions, or CDRs, of the antibody or antigen-binding portion thereof of the disclosure. The nucleic acid molecule can be present in whole cells, in a cell lysate, or in a partially purified or substantially pure form. A nucleic acid molecule is "isolated" or "rendered substantially pure" when purified away from other cellular components or other contaminants, e.g., other cellular nucleic acids or proteins, by standard techniques. A nucleic acid molecule of the disclosure can be, e.g., DNA or RNA and may or may not contain intronic sequences. In a preferred embodiment, the nucleic acid molecule is a cDNA molecule.

The nucleic acid molecule of the disclosure can be obtained using standard molecular biology techniques. For antibodies expressed by hybridomas (e.g., hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (e.g., using phage display techniques), a nucleic acid molecule encoding such antibodies can be recovered from the gene library.

Preferred nucleic acids molecules of the disclosure include those encoding the V_{H} and V_{L} sequences of the B7-H3 monoclonal antibody or the CDRs. Once DNA fragments encoding V_{H} and V_{L} segments are obtained, these DNA fragments can be further manipulated by standard recombinant DNA techniques, for example to convert the variable region genes to full-length antibody chain genes, to Fab fragment genes or to a scFv gene. In these manipulations, a V_{L}- or V_{H}-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the V_{H} region can be converted to a full-length heavy chain gene by operatively linking the V_{H}-encoding DNA to another DNA molecule encoding heavy chain constant regions (C_{H1}, C_{H2} and C_{H3}). The sequences of human heavy chain constant region genes are known in the art and DNA fragments encompassing these regions can be obtained by standard PCR amplification. The heavy chain constant region can be an IgG1, IgG2, IgG3, IgG4, IgA, IgE, IgM or IgD constant region, but preferably an IgG1 or IgG4 constant region. For a Fab fragment heavy chain gene, the V_{H}-encoding DNA can be operatively linked to another DNA molecule encoding only the heavy chain C_{H1} constant region.

The isolated DNA encoding the V_{L} region can be converted to a full-length light chain gene by operatively linking the V_{L}-encoding DNA to another DNA molecule encoding the light chain constant region, C_{L}. The sequences of human light chain constant region genes are known in the art and DNA fragments encompassing these regions can be obtained by standard PCR amplification. In preferred embodiments, the light chain constant region can be a κ or λ constant region.

To create a scFv gene, the V_{H}- and V_{L}-encoding DNA fragments are operatively linked to another fragment encoding a flexible linker, such that the V_{H} and V_{L} sequences can be expressed as a contiguous single-chain protein, with the V_{L} and V_{H} regions joined by the flexible linker (see e.g., Bird et al., (1988) Science 242:423-426; Huston et al., (1988) Proc. Natl. Acad. Sci. USA 85:5879-5883; McCafferty et al., (1990) Nature 348:552-554).

The monoclonal antibody of the disclosure may be prepared using the somatic cell hybridization (hybridoma) technology as described in Kohler and Milstein (1975) Nature 256: 495. Other embodiments for preparing monoclonal antibodies include virus or carcinogenesis-mediated B lymphocyte transformation, and phage display technology. The chimeric or humanized antibodies are well known in the art. See, e.g., US patent 4,816,567; 5,225,539; 5,530,101; 5,585,089; 5,693,762 and 6,180,370.

Antibodies of the disclosure also can be produced in a host cell transfectoma using, for example, a combination of recombinant DNA techniques and gene transfection methods as is well known in the art (e.g., Morrison, S. (1985) Science 229:1202). In one embodiment, DNA encoding partial or full-length light and heavy chains obtained by standard molecular biology techniques is inserted into one or more expression vectors such that the genes are operatively linked to transcriptional and translational regulatory sequences. In this context, the term "operatively linked" is intended to mean that an antibody gene is ligated into a vector such that transcriptional and translational control sequences within the vector serve their intended function of regulating the transcription and translation of the antibody gene.

The term "regulatory sequence" is intended to include promoters, enhancers and other expression control elements (e.g., polyadenylation signals) that control the transcription or translation of the antibody genes. Such regulatory sequences are described, e.g., in Goeddel (Gene Expression Technology. Methods in Enzymology 185, Academic Press, San Diego, Calif. (1990)). Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, e.g., the adenovirus major late promoter (AdMLP) and polyomavirus enhancer. Alternatively, non-viral regulatory sequences can be used, such as the ubiquitin promoter or β-globin promoter. Still further, regulatory elements composed of sequences from different sources, such as the SRα promoter system, which contains sequences from the SV40 early promoter and the long terminal repeat of human T cell leukemia virus type 1 (Takebe et al., (1988) Mol. Cell. Biol. 8:466-472). The expression vector and expression control sequences are chosen to be compatible with the expression host cell used.

The antibody light chain gene and the antibody heavy chain gene can be inserted into the same or separate expression vectors. In preferred embodiments, the variable regions are used to create full-length antibody genes of any antibody isotype by inserting them into expression vectors already encoding heavy chain constant and light chain constant regions of the desired isotype such that the V_{H} segment is operatively linked to the C_{H} segment(s) within the vector and the V_{L} segment is operatively linked to the C_{L} segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain genes and regulatory sequences, the recombinant expression vectors of the disclosure can carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216; 4,634,665 and 5,179,017). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr-host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies of the disclosure in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody.

Preferred mammalian host cells for expressing the recombinant antibodies of the disclosure include, the Slc35C1 knock-out cell line, the FUT8 knock-out cell line, the Lec13 cell line (variant CHO cell line), the rat myeloma cell line YB2/0, the cell line containing small interfering RNAs specifically against gene fut8, and the cell line co-expressing β-1,4-N-acetylglucosaminyltransferase III and Golgi α-mannosidase II, Chinese Hamster Ovary (CHO cells) (including dhfr-CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) J. Mol. Biol. 159:601-621), NSO myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

The antibody or antigen binding portion thereof of the disclosure can be conjugated to a therapeutic agent to form an immunoconjugate such as an antibody-drug conjugate (ADC). Suitable therapeutic agents include cytotoxins, alkylating agents, DNA minor groove binders, DNA intercalators, DNA crosslinkers, histone deacetylase inhibitors, nuclear export inhibitors, proteasome inhibitors, topoisomerase I or II inhibitors, heat shock protein inhibitors, tyrosine kinase inhibitors, antibiotics, and anti-mitotic agents. In the ADC, the antibody and therapeutic agent preferably are conjugated via a linker cleavable such as a peptidyl, disulfide, or hydrazone linker. More preferably, the linker is a peptidyl linker such as Val-Cit, Ala-Val, Val-Ala-Val, Lys-Lys, Ala-Asn-Val, Val-Leu-Lys, Ala-Ala-Asn, Cit-Cit, Val-Lys, Lys, Cit, Ser or Glu. The ADCs can be prepared as described in U.S. Pat. Nos. 7,087,600; 6,989,452; and 7,129,261; PCT Publications WO 02/096910; WO 07/038,658; WO 07/051,081; WO 07/059,404; WO 08/083,312; and WO 08/103,693; U.S. Patent Publications 20060024317; 20060004081; and 20060247295.

In another aspect, the present disclosure features a bispecific molecule which may comprise one or more antibodies of the disclosure linked to at least one other functional molecule, e.g., another peptide or protein (e.g., another antibody or ligand for a receptor) to generate a bispecific molecule that binds to at least two different binding sites or target molecules. Thus, as used herein, "bispecific molecule" includes molecules that have three or more specificities.

Bispecific molecules may be in many different formats and sizes. At one end of the size spectrum, a bispecific molecule retains the traditional antibody format, except that, instead of having two binding arms of identical specificity, it has two binding arms each having a different specificity. At the other extreme are bispecific molecules consisting of two single-chain antibody fragments (scFv's) linked by a peptide chain, a so-called Bs(scFv)₂ construct. Intermediate-sized bispecific molecules include two different F(ab) fragments linked by a peptidyl linker. Bispecific molecules of these and other formats can be prepared by genetic engineering, somatic hybridization, or chemical methods. See, e.g., Kufer *et al,* cited supra; Cao and Suresh, Bioconjugate Chemistry, 9 (6), 635-644 (1998); and van Spriel et al., Immunology Today, 21 (8), 391-397 (2000).

Also provided herein are a chimeric antigen receptor containing an anti-B7-H3 scFv, the anti-B7-H3 scFv comprising CDRs and heavy/light chain variable regions described herein.

The anti-B7-H3 chimeric antigen receptor may comprise (a) an extracellular antigen binding domain comprising an anti-B7-H3 scFv; (b) a transmembrane domain; and (c) an intracellular signaling domain.

An oncolytic virus preferentially infects and kills cancer cells. The antibody or antigen binding portion thereof of the disclosure may be used in conjunction with the oncolytic virus. Alternatively, an oncolytic virus encoding the antibody or antigen binding portion thereof of the disclosure can be introduced into human body.

In another aspect, the present disclosure provides a pharmaceutical composition comprising one or more antibodies or antigen binding portions thereof, the antibody or antigen binding portion-encoding vectors, immunoconjugates, immune cells, bi-specific antibodies, and/or oncolytic viruses, of the disclosure, formulated together with a pharmaceutically acceptable carrier. The composition may optionally contain one or more additional pharmaceutically active ingredients, such as an anti-tumor antibody, or an antibody for immunity enhancement, or a non-antibody anti-tumor agent or immunity enhancement agent. The pharmaceutical composition of the disclosure may be used in combination with, for example, an anti-tumor agent, or an agent for immunity enhancement.

The pharmaceutical composition may comprise any number of excipients. Excipients that can be used include carriers, surface active agents, thickening or emulsifying agents, solid binders, dispersion or suspension aids, solubilizers, colorants, flavoring agents, coatings, disintegrating agents, lubricants, sweeteners, preservatives, isotonic agents, and combinations thereof. The selection and use of suitable excipients are taught in Gennaro, ed., Remington: The Science and Practice of Pharmacy, 20th Ed. (Lippincott Williams & Wilkins 2003).

Preferably, the pharmaceutical composition is suitable for intravenous, intramuscular, subcutaneous, parenteral, spinal or epidermal administration (e.g., by injection or infusion). Depending on the route of administration, the active ingredient can be coated in a material to protect it from the action of acids and other natural conditions that may inactivate it. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion. Alternatively, an antibody of the disclosure can be administered via a non-parenteral route, such as a topical, epidermal or mucosal route of administration, e.g., intranasally, orally, vaginally, rectally, sublingually or topically.

Pharmaceutical compositions can be in the form of sterile aqueous solutions or dispersions. They can also be formulated in a microemulsion, liposome, or other ordered structure suitable to high drug concentration.

The amount of active ingredient which can be combined with a carrier material to produce a single dosage form will vary depending upon the subject being treated and the particular mode of administration and will generally be that amount of the composition which produces a therapeutic effect. Generally, out of one hundred percent, this amount will range from about 0.01% to about 99% of active ingredient in combination with a pharmaceutically acceptable carrier.

Dosage regimens are adjusted to provide the optimum desired response (e.g., a therapeutic response). For example, a single bolus can be administered, several divided doses can be administered over time or the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active ingredient calculated to produce the desired therapeutic effect in association with the required pharmaceutical carrier. Alternatively, the antibody can be administered as a sustained release formulation, in which case less frequent administration is required.

For administration of the antibody, the dosage may range from about 0.001 to 100 mg/kg. An exemplary treatment regime entails administration once per week. The preferable way of anti-B7-H3 administration includes intravenous administration.

A "therapeutically effective dosage" of the anti-B7-H3 antibody of the disclosure results in a decrease in severity of disease symptoms, an increase in frequency and duration of disease symptom-free periods. For example, for the treatment of tumor-bearing subjects, a "therapeutically effective dosage" preferably inhibits tumor growth by at least about 20%, more preferably by at least about 40%, even more preferably by at least about 60%, and still more preferably by at least about 80% relative to untreated subjects. An effective amount of the therapeutic antibody may reduce tumor size, or alleviate the subject's symptoms. The subject may be human or another mammalian animal.

The pharmaceutical composition can be a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid. See, e.g., Sustained and Controlled Release Drug Delivery Systems, J. R. Robinson, ed., Marcel Dekker, Inc., New York, 1978.

The pharmaceutical compositions can be administered via medical devices such as (1) needleless hypodermic injection devices (e.g., U.S. Pat. Nos. 5,399,163; 5,383,851; 5,312,335; 5,064,413; 4,941,880; 4,790,824; and 4,596,556); (2) micro-infusion pumps (U.S. Pat. No. 4,487,603); (3) transdermal devices (U.S. Pat. No. 4,486,194); (4) infusion apparatuses (U.S. Pat. Nos. 4,447,233 and 4,447,224); and (5) osmotic devices (U.S. Pat. Nos. 4,439,196 and 4,475,196); the disclosures of which are incorporated herein by reference.

In certain embodiments, the antibodies of the disclosure can be formulated to ensure proper distribution *in vivo.* For example, to ensure that the therapeutic antibody of the disclosure cross the blood-brain barrier, they can be formulated in liposomes, which may additionally comprise targeting moieties to enhance selective transport to specific cells or organs. See, e.g. U.S. Pat. Nos. 4,522,811; 5,374,548; 5,416,016; and 5,399,331; V. V. Ranade (1989) J. Clin.Pharmacol.29:685*;* Umezawa et al., (1988) Biochem. Biophys. Res. Commun. 153:1038; Bloeman et al., (1995) FEBS Lett.357:140; M. Owais et al., (1995) Antimicrob. Agents Chemother. 39:180; Briscoe et al., (1995) Am. J. Physiol. 1233:134; Schreier et al., (1994) J. Biol. Chem. 269:9090; Keinanen and Laukkanen (1994) FEBS Lett. 346:123; and Killion and Fidler (1994) Immunomethods 4:273.

The pharmaceutical composition of the present disclosure may have numerous *in vitro* and *in vivo* utilities involving, for example, cancer treatment, or more generally the enhancement of the immune system in patients having diseases such as cancers. The pharmaceutical composition may be administered to human subjects, to e.g., *in vivo* inhibit tumor growth.

Given the ability of the pharmaceutical composition of the disclosure to inhibit tumor cell proliferation and survival, the present application provides a method for inhibiting tumor cell growth in a subject, comprising administering to the subject the pharmaceutical composition of the disclosure, so that the tumor growth is inhibited in the subject. The non-limiting examples of the tumor that can be treated by the antibodies of the disclosure include, but not limited to, breast cancer, melanoma, prostate cancer, head and neck squamous cell carcinoma, lung cancer, non-small cell lung cancer, central nervous system neuroblastoma, glioblastoma, desmoplastic small round cell tumor, diffuse Pontine glioma, medulloblastoma, pancreatic cancer, liver cancer, colorectal cancer, non-Hodgkin lymphoma, esophageal cancer, ovarian cancer, bladder cancer, small cell carcinoma, endometrial cancer, kidney cancer, gastric cancer, acute myeloid leukemia, hepatocellular carcinoma, hypopharyngeal squamous cell carcinoma, urothelial cell carcinoma, original or metastatic. Refractory or recurrent malignancies may also be treated by the pharmaceutical composition of the disclosure. The pharmaceutical composition of the disclosure may also treat autoimmune diseases, such as asthma.

The pharmaceutical composition of the disclosure may be used to enhance an immune response in a subject, to e.g., activate immune cells such as NK cells and T cells.

The present application provides a combination therapy where the pharmaceutical composition of the disclosure is administered with one or more other antibodies or non-antibody therapeutic agents, which can effectively inhibit tumor growth in subjects. In one embodiment, the present application provides a method for inhibiting tumor growth in a subject, comprising administering to the subject the pharmaceutical composition of the disclosure and one or more other antibodies, such as an anti-PD-1 antibody, an anti-PD-L1 antibody and/or an anti-4-1BB antibody. In certain embodiments, the subject is human. In another aspect, the present application provides a cancer treatment method where the pharmaceutical composition of the disclosure is administered with a chemotherapeutic agent, wherein the chemotherapeutic agent may be a cytotoxic agent. Other therapies that can be combined with the pharmaceutical composition of the disclosure, include, but not limited to, administration of an immunogenic agent, administration of interleukine-2 (IL-2), radiotherapy, surgery, or hormone deprivation.

The combination of therapeutic agents discussed herein can be administered concurrently as a single composition in a pharmaceutically acceptable carrier, or concurrently as separate compositions with each agent in a pharmaceutically acceptable carrier. In another embodiment, the combination of therapeutic agents can be administered sequentially.

Furthermore, if more than one dose of the combination therapy is administered sequentially, the order of the sequential administration can be reversed or kept in the same order at each time point of administration, sequential administrations can be combined with concurrent administrations, or any combination thereof.

The present disclosure is further illustrated by the following examples, which should not be construed as further limiting. The figures and all references, Genbank sequences, patents and published patent applications cited throughout this application are expressly incorporated herein by reference in their entirety.

### Examples

### Example 1. Generation of anti-human B7-H3 monoclonal antibodies

To generate anti-human B7-H3 antibodies, B7-H3 RenMab KO mice were immunized with a recombinant human B7-H3 (4Ig) protein (human B7-H3 (4Ig)/B7-H3b protein, with His tag, Cat#: B7B-H52E7, ACROBiosystems) and/or a recombinant human B7-H3 (2Ig) protein (human B7-H3/CD276 protein, with His tag (MALS verified), Cat#: B73-H52E2, ACROBiosystems). The RenMab mice contained a gene locus for humanized immunoglobulin heavy chain and a gene locus for humanized immunoglobulin κ chain. The gene locus for the immunoglobulin heavy chain contained a region with the gene encoding the antibody heavy chain, and this gene locus contained the genes for IGHV (variable), IGHD (diversity), IGHJ (joining) and heavy chain constant region. The gene locus for the immunoglobulin κ chain contained the gene for coding the antibody light chain (κ chain). The gene locus for the immunoglobulin κ chain contained the genes for IGKV (variable), IGKJ (joining) and light chain constant region. The detailed description of the RenMab mice can be found in PCT/CN2020/075698, which is incorporated herein by reference in its entirety. The B7-H3 RenMab KO mice were obtained by knocking out the B7-H3 gene of the RenMab mice and did not express functional B7-H3 proteins.

### Mice immunization

Four rounds of immunization were performed in B7-H3 RenMab KO mice using the recombinant human B7-H3 (4Ig) protein. The protein was mixed with an adjuvant in a 1:1 v/v ratio, and then emulsified. Each animal was injected at the heel and the neck. The adjuvant used for the first immunization was Complete Freund's Adjuvant (CFA), and the adjuvant for the 2^{nd} to 4^{th} immunizations was Incomplete Freund's Adjuvant (IFA). Two injections should be at least 14 days apart.

In another test that was run in parallel, the immunization was done by alternatively using the recombinant human B7-H3 (4Ig) protein and the recombinant human B7-H3 (2Ig) protein. The proteins and the adjuvant were mixed in a 1:1 v/v ratio and then emulsified. Each animal was injected at the heel and the neck. The adjuvant used for the first immunization was Complete Freund's Adjuvant (CFA), and the adjuvant for the 2^{nd} to 4^{th} immunizations was Incomplete Freund's Adjuvant (IFA). Two injections should be at least 14 days apart.

The peripheral bloods (serums) were collected, and the antibody titers were measured by fluorescence-activated cell sorting (FACS) or ELISA.

The last boost immunization was done at least 14 days post the 4^{th} immunization by intraperitoneal injection of the B7-H3 (4Ig) protein and the tail vein injection of CHO cells expressing on the surfaces the B7-H3 antigens.

### Antibody discovery

Four days after the last immunization, the mice were sacrificed. The B cells positive for the antigens were isolated directly from inside the mice, and subjected to the single cell sorting (Beacon^{®} Optofluidic System, Berkeley Lights Inc.) to obtain plasmas cells secreting antigen-specific monoclonal antibodies. The antibody variable region sequences were obtained by reverse transcription and PCR, and the antibody heavy/light chain variable regions were cloned to the vectors coding for the human IgG constant region and human κ constant region, to express antibodies, which were tested for their binding specificity to B7-H3 by FACS. The exemplary fully human antibodies as obtained included 6B5, 10D7, 10F5, 10F7 and 15F11, the heavy and light chain variable region and CDR sequences as well as the SEQ ID NO were set forth in Table 1 and Table 9.

### Antibody construction, expression and purification

The fragments encoding variable regions and human IgG1/κ constant regions (the amino acid sequences of the heavy chain constant region and light chain constant region set forth in SEQ ID NOs: 43 and 45, respectively) were inserted between the restriction sites XhoI/BamHI in pCDNA3.1 (Invitrogen, US) to construct antibody expression vectors.

The expression vectors as obtained above were used to transfect HEK-293F cells (Cobioer, CN) with PEI. Particularly, the HEK-293F cells were cultured in Free Style^{™} 293 expression medium (Cat#: 12338-018, Gibco, US), and each expression vector was transfected to the cells with polyethylenimine (PEI), the ratio of DNA to PEI being 1:3, wherein the amount of DNA was 1.5 µg per milliliter cell culture medium. The HEK-293F cells after transfection were cultured in a 37°C, 5%CO₂ incubator shaker at 120 rpm. The cell culture supernatants were collected after 10 to 12 days, centrifuged at 3500 rpm for 5 min, and filtered through a 0.22 µm membrane to remove cell debris. The monoclonal antibodies were enriched and purified by passing through a pre-balanced Protein A affinity column (Cat#: 17040501, GE, US), and eluted using an elution buffer (20 mM citric acid, pH3.0-pH3.5). Thereafter, the antibodies were stored in PBS (pH 7.0), and measured for antibody concentration in a NanoDrop spectrophotometer.

### Example 2. Affinity measurement of fully human anti-B7-H3 antibodies

The fully human anti-human B7-H3 antibodies were tested for their binding affinity to human B7-H3 (4Ig), human B7-H3 (2Ig), monkey B7-H3 (monkey B7-H3/CD276 protein, with His tag, Cat#: B73-C52Ha, ACROBiosystems) and mouse B7-H3 protein (mouse B7-H3/CD276 protein, with His tag, Cat#: B73-M52H4, ACROBiosystems) by surface plasmon resonance (SPR) using Biacore T200 biosensor (Biacore, US) provided with a pre-fixed Protein A sensor chip. Briefly, the anti-human B7-H3 antibodies (1 µg/mL) were injected to the Biacore T200 biosensor at 10 µL/min for 20 sec, to achieve the desired protein density (100 ± 30 RU). Then, the antigen proteins, at the concentration of 200 nM, 100 nM, 50 nM, 25 nM, 12.5 nM, 6.25 nM, 3.125 nM, 1.5625 nM, 0.78125 nM and 0 nM, were injected at 30 µL/min for 180 sec, and the dissociation was monitored for 600 sec. Enoblituzumab, a humanized anti-B7-H3 antibody developed by MacroGenics with Fc optimization (see US8802091B2 for antibody sequences, the heavy and light chain variable region sequences set forth in SEQ ID NOs: 39 and 40, respectively, the heavy chain constant region was an IgG1 constant region with 5 point mutations F243L/R292P/Y300L/L235V/P396L to enhance ADCC, the light chain constant region was a κ constant region, with the sequences set forth in SEQ ID NOs: 44 and 45, respectively) was used as the positive control.

The data as generated was analyzed using Biacore Insight V2.0.1 5.12933 with the one-to-one Langmuir binding model. The association rate (kₒₙ) and dissociation rate (k_{off}) were obtained at the same time. The affinity was calculated as the quotient of the two kinetic rate constants (K_{D} = k_{off}/kₒₙ).

All the antibodies of the disclosure bound to the two splicing variants of human B7-H3, and also the monkey B7-H3 protein. The antibodies 6B5 and 15F11 also bound to the mouse B7-H3 protein. The affinity test results of the representative antibodies of the disclosure were shown in Table 2 and Table 3.

**Table 2. Binding affinity of anti-B7-H3 antibodies to human B7-H3 (4Ig) and human B7-H3 (2Ig)**

| Antibody | Human B7-H3 (4Ig) | | | Human B7-H3 (2Ig) | | |
|---|---|---|---|---|---|---|
| | Ka (1/Ms) | Kd (1/s) | K_{D} (M) | Ka (1/Ms) | Kd (1/s) | K_{D} (M) |
| 6B5 | 2.47E+05 | 3.10E-04 | 1.26E-09 | 1.27E+05 | 6.53E-03 | 5.15E-08 |
| 10D7 | 2.63E+05 | 2.91E-04 | 1.11E-09 | 1.52E+05 | 5.88E-02 | 3.86E-07 |
| 10F5 | 1.09E+05 | 1.10E-04 | 1.01E-09 | 4.05E+04 | 4.22E-04 | 1.04E-08 |
| 10F7 | 2.25E+05 | 5.57E-04 | 2.48E-09 | 7.37E+04 | 2.03E-02 | 2.75E-07 |
| 15F11 | 8.51E+04 | 5.10E-04 | 5.99E-09 | 3.43E+03 | 2.48E-02 | 7.24E-06 |
| Enoblituzumab | 2.16E+05 | 2.39E-04 | 1.11E-09 | 7.37E+04 | 2.03E-02 | 2.75E-07 |

**Table 3. Binding affinity of anti-B7-H3 antibodies to monkey B7-H3 and mouse B7-H3**

| Antibody | Monkey B7-H3 | | | Mouse B7-H3 | | |
|---|---|---|---|---|---|---|
| | Ka (1/Ms) | Kd (1/s) | K_{D} (M) | Ka (1/Ms) | Kd (1/s) | K_{D} (M) |
| 6B5 | 2.58E+05 | 2.95E-04 | 1.14E-09 | 5.83E+04 | 1.59E-02 | 2.74E-07 |
| 10D7 | 3.37E+05 | 1.67E-02 | 4.95E-08 | No binding | No binding | No binding |
| 10F7 | 2.92E+05 | 5.13E-04 | 1.76E-09 | / | / | 3.70E-07 |
| 15F11 | 1.15E+05 | 4.74E-04 | 4.12E-09 | 1.40E+03 | 1.09E-03 | 7.81E-07 |
| Enoblituzumab | 2.31E+05 | 2.23E-04 | 9.66E-10 | No binding | No binding | No binding |

### Example 3. Binding activity of fully human anti-B7-H3 antibodies to B7-H3⁺ tumor cells, B7-H3⁻ tumor cells and B7-H3⁺ normal cells

To determine the binding activity of the fully human anti-B7-H3 antibodies of the disclosure to different types of B7-H3⁺ tumor cells, B7-H3⁻ tumor cells and B7-H3⁺ normal cells, FACS was performed using A549 cells (Cat#: CBP60084, COBIOER), NCI-H520 cells (Cat#: CBP60139, COBIOER), HepG2 cells (Cat#: CBP60199, COBIOER), SK-OV-3 cells (Cat#: CBP60291, COBIOER) and MCF-7 cells (Cat#: CL-0149, ProCell) as the representative B7-H3⁺ tumor cells, Jurkat cells (Cat#: CBP60520, COBIOER), Daudi cells (Cat#: CBP60262, COBIOER), and Raji cells (Cat#: CBP60272, COBIOER) as the representative B7-H3⁻ tumor cells (according to literatures, these three cells were B7-H3 mRNA negative), and human prostate smooth muscle cells (Cat#: CC-2587, Lonza), human dermal fibroblasts (Cat#: CC-2511, Lonza), human aorta smooth muscle cells (Cat#: H-6080, Cell Biologics) and human dendritic cells (from human PBMC) as the representative normal cells.

The A549 cells, NCI-H520 cells, Jurkat cells, Daudi cells, and Raji cells, were cultured in RPMI1640 medium (Cat#: 12-115F, Lonza) supplemented with 10% FBS (Cat#: FND500, Excell), and 1% penicillin-streptomycin (Cat#: SV30010, Hyclone). The HepG2 cells and SK-OV-3 cells were respectively cultured in MEM medium (Cat#: 12561-056, Gibco) and McCoy's 5A medium (Cat#: 16600-082, Hyclone), both supplemented with 10% FBS (Cat#: FND500, Excell) and 1% penicillin-streptomycin (Cat#: SV30010, Hyclone). The MCF-7 cells were cultured in MCF7 cell specialized medium (Cat#: CM-0149, ProCell), and the human prostate smooth muscle cells were cultured in SmGM^{™}-2 smooth muscle cell growth medium BulletKit^{™} (Cat#: CC-3182, Lonza) according to the manual. The human dermal fibroblasts were cultured in FGM^{™}-2 fibroblast growth medium BulletKit^{™} (Cat#: CC-3132, Lonza) according to the manual, and human aorta smooth muscle cells were cultured in Complete smooth muscle cell medium (Cat#: M2268, Cell Biologics) according to the manual.

The human dendritic cells were obtained by isolating PBMCs followed by *in vitro* induction. Briefly, the PBMCs (Cat#: PB004F-C, ALLCELLS) were re-suspended in RPMI1640 medium and incubated in a 37°C incubator for 2 hr. The cells adhered to the walls, i.e., the isolated monocytes, were collected. The monocytes were cultured in RPMI1640 medium supplemented with 100 ng/ml recombinant human GM-CSF (Cat#: 7954-GM, R&D), 100 ng/ml recombinant human IL-4 (Cat#: 6507-IL, R&D) and 10% FBS. After 3 days, half of the culture medium was replaced with fresh one. On Day 6 of the culture, the culture medium was replaced with the medium containing 100 ng/ml recombinant human GM-CSF, 100 ng/ml recombinant human IL-4, 10 ng/ml rhTNF-α (Cat#: 210-TA-100, R&D), 1000 U/ml rhIL-6 (Cat#: 7270-IL-025, R&D), 1 µg/ml PGE2 (Cat#: 363-24-6, TOCRIS) and 10 ng/ml IL-1β (Cat#: 210-LB-025, R&D). After another two days of cell incubation, mature human dendritic cells were obtained.

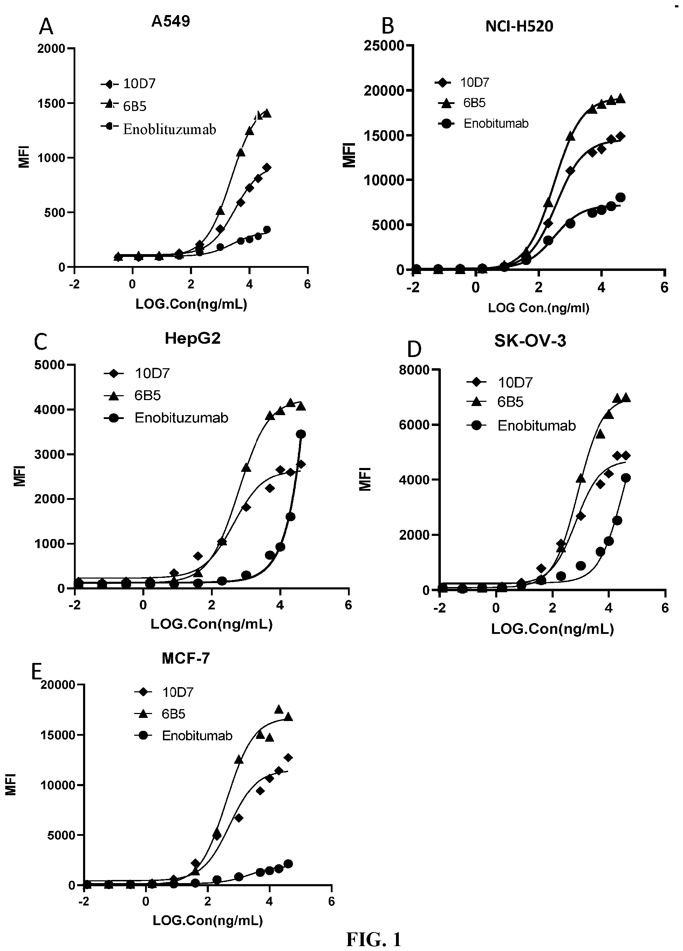

The anti-B7-H3 antibodies were tested for their binding activity to the cells above. Briefly, 10⁵ cells in 100 µl culture medium were plated onto 96-well plates, and added with 50 µl serially diluted anti-B7-H3 antibodies. After incubation at 4°C for 1 hr, the 96-well plates were washed with PBST for 3 times. Then, the plates were added with 1:500 diluted PE-goat-anti-mouse IgG (Cat#: PA1-86078, Invitrogen). After incubation at 4°C for 1 hr, the 96-well plates were washed with PBS for 3 times, and then subjected to cell fluorescence measurement using a FACS machine (BD). Enoblituzumab was used as the positive control. The test results of the representative anti-B7-H3 antibodies of the disclosure were shown in FIG. 1 to FIG. 3.

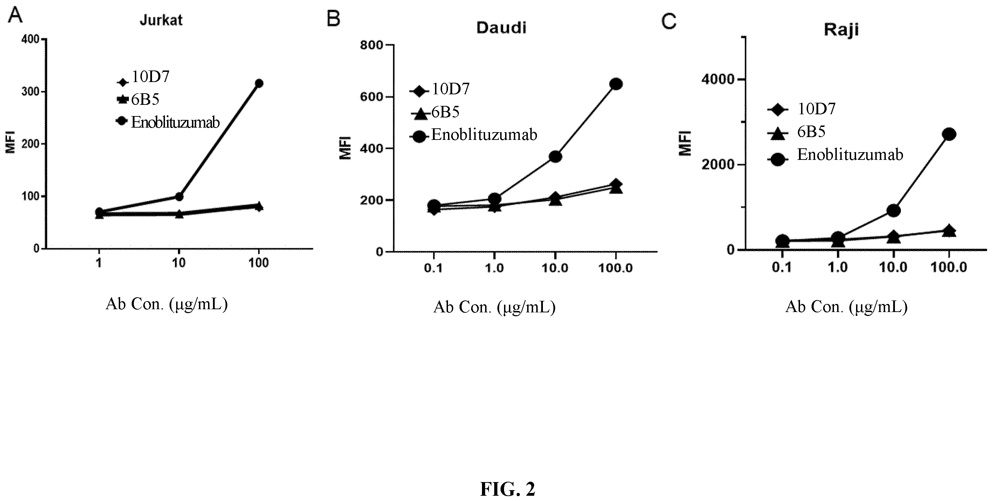

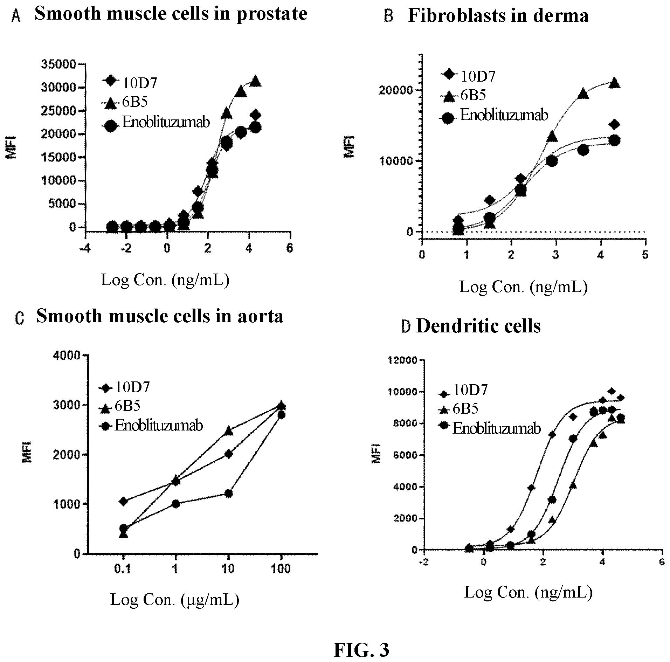

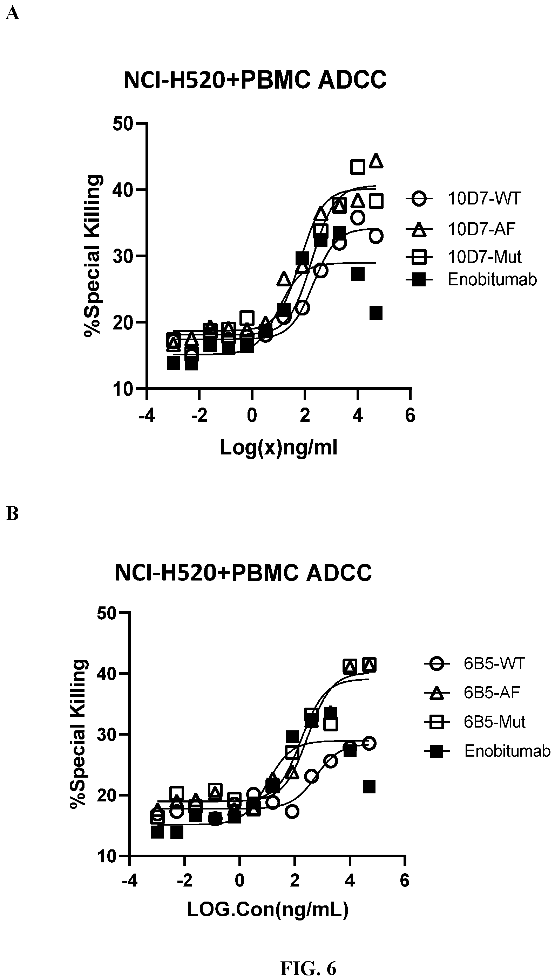

As shown in FIG. 1, 6B5 and 10D7 bound to various types of B7-H3⁺ tumor cells, with the binding activity both significantly higher than that of enoblituzumab. It can be seen from FIG. 2 that there was almost no binding of 6B5 or 10D7 to the B7-H3 mRNA⁻ tumor cells, and the binding activity was significantly lower than that of enoblituzumab. All these suggested that the antibodies of the disclosure had better B7-H3 binding specificity. As shown in FIG. 3, 6B5's binding ability to several normal human B7-H3⁺ cells was a bit higher than enoblituzumab, while 10D7's binding activity was comparable to enoblituzumab's.

### Example 4. Fc engineering did not affect fully human anti-B7-H3 antibodies' binding to B7-H3⁺ cells

The fragments encoding the heavy chain/light chain variable regions of 10D7 and 6B5 plus human IgG1/κ constant regions (SEQ ID NOs: 43 and 45) were cloned into pCDNA3.1 (Invitrogen, US), to construct the expression vectors which were transfected into CHO-K1 cells and slc35c1 knock-out CHO-K1-AF cells. The slc35c1 knock-out CHO-K1-AF cells were generated by Beijing MabWorks Biotech Inc., the detailed generation method can be found in US10377833B2, and the proteins expressed by such cell line was basically with no fucosylation. The 10D7 and 6B5 were transiently expressed in CHO-K1 and CHO-K1-AF cells and purified, the obtained antibodies were designated as 10D7-WT, 6B5-WT, 10D7-AF (afucosylated) and 6B5-AF (afucosylated). In the meanwhile, the fragments encoding the heavy chain/light chain variable regions of 10D7 and 6B5 plus 5-point-mutated human IgG1 Fc/κ constant regions (SEQ ID NOs: 44 (L235V, F243L, R292P, Y300L and P396L, according to EU numbering) and 45), were cloned into pCDNA3.1 to construct the expression vectors which were transfected into CHO-K1 cells for transient expression, the obtained antibodies were designated as 10D7-Mut and 6B5-Mut. The point mutations in the Fc region and the afucosylation were used to enhance ADCC. The antibody expression and purification were performed according to the protocol in Example 1.

These anti-B7-H3 antibodies were tested for their binding ability to human B7-H3-expressing HUVEC cells (Cat#: C-12200, PromoCell, Germany) and NCI-H520 cells by FACS. The HUVEC cells were cultured in EGM^{™}-2 Endothelial Cell Growth Medium-2 BulletKit^{™} (Cat#: CC-3162, Lonza), and subject to passage according to the manual. See Example 3 for the details of NCI-H520 cell culture and FACS test.

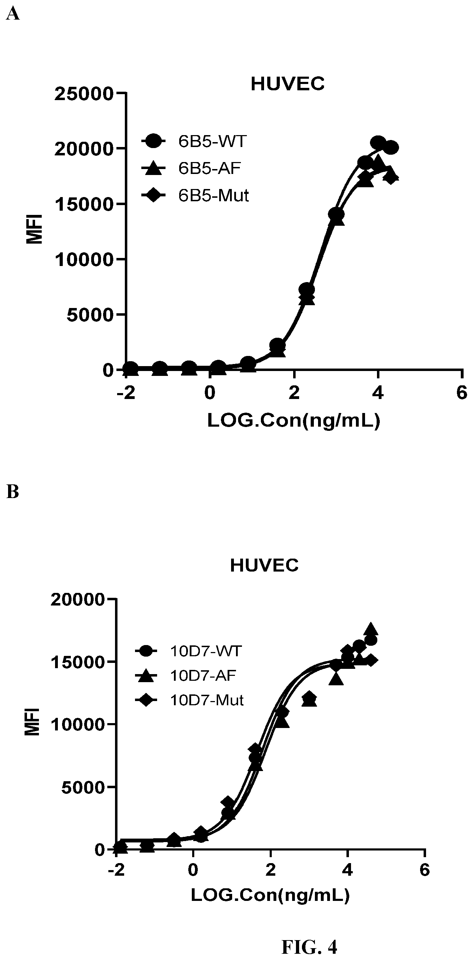

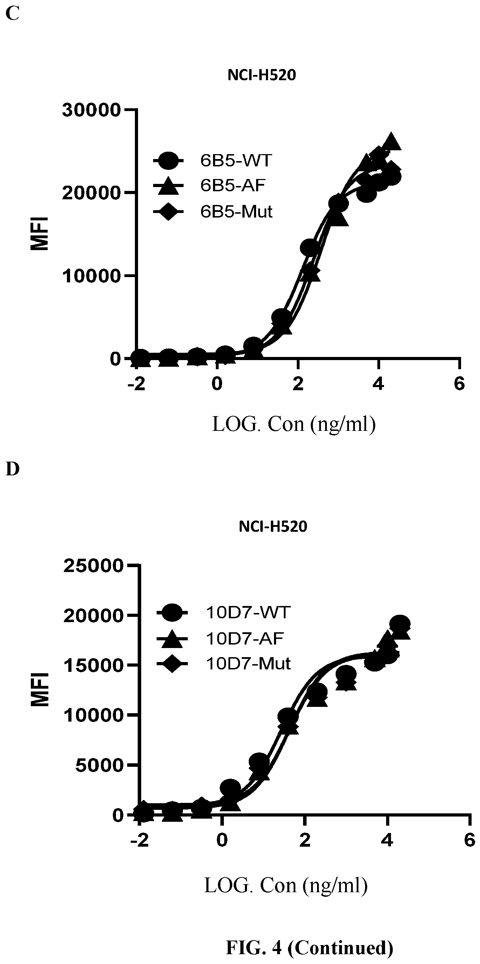

As shown in FIG. 4, the change in Fc region, afucosylation or point mutations, did not affect the binding of the anti-B7-H3 antibodies to the B7-H3⁺ cells. The 10D7-AF, 10D7-WT and 10D7-Mut showed completely the same binding activity to HUVEC (B) and NCI-H520 cells (D), and 6B5-AF, 6B5-WT and 6B5-Mut showed almost the same binding activity to HUVEC (A) and NCI-H520 cells (C).

### Example 5. Fully human anti-B7-H3 antibodies' activity to induce ADCC and to activate immune cells

The anti-B7-H3 antibodies of the disclosure were further tested for their ability to induce antibody dependent cytotoxicity (ADCC) against B7-H3⁺ NCI-H520 tumor cells, B7-H3⁺ human umbilical vein endothelial cells (HUVEC) and mature dendritic cells (DC), and to activate NK cells and PBMCs, wherein the dendritic cells were induced, differentiated and maturated from the isolated PBMCs (see details in Example 3).

Briefly, the NCI-520 cells, HUVEC cells and DCs were re-suspended in the complete RPMI culture medium at the cell density of 1.0×10⁶/ml. The cells were labelled by carboxyfluorescein succinimidyl ester (CFSE, Cat#: C34554I, Invitrogen). Specifically, the cells were incubated with 2.5 µM CFSE at 37°C for 10 min. After labelling, the cells were re-suspended in complete RPMI culture medium (RPMI+10%FBS). The NCI-H520 cells, HUVEC cells, DCs and NK92MI-CD16a (as the effector cells, Huabo Bio) were centrifuged at 1200 rpm for 5 min. These cells were suspended in the ADCC test medium (MEM medium, Cat#: 12561-056, Gibco; 1% FBS, Cat#: FND500, EX-cell; 1% BSA, Cat#: V900933-1KG, VETEC), and the cell viability was about 90% according to the cell counting results. Fifty µl of the target cells (NCI-H520 cells, HUVEC cells or DCs) at the cell density of 4×10⁵/ml and 50 µl of the NK92MI-CD16a cells at the cell density of 2×10⁶/ml were added to each well of the 96-well plates, the effector: target ratio being 5:1. The antibodies to be tested were added to each well at different concentrations, such that the starting final concentration was 50000 ng/ml and the other final concentrations were 5-fold diluted, 10 concentrations in total. The 96-well plates were incubated at 37°C for 4 hr, and the cells on the plates were washed with PBS for 3 times, and then incubated with LIVE/DEAD fixable dead cell stain at 37°C for 30 min. The cells on the plates were washed with PBS for 3 times, and added with 2 µl PE-mouse anti-human CD69 antibody (Cat#: 555531, BD). After incubation at ambient temperature for 30 min, the plates were centrifuged, washed with PBS for 3 times, and subject to FACS. The cell death rate of the CFSE⁺ cells (NCI-H520 cells, HUVEC cells or DCs) was determined, and the average PE staining fluorescent intensity of the CFSE⁻cells (NK92MI-CD16a cells) were calculated. Enoblituzumab was used as the positive control.

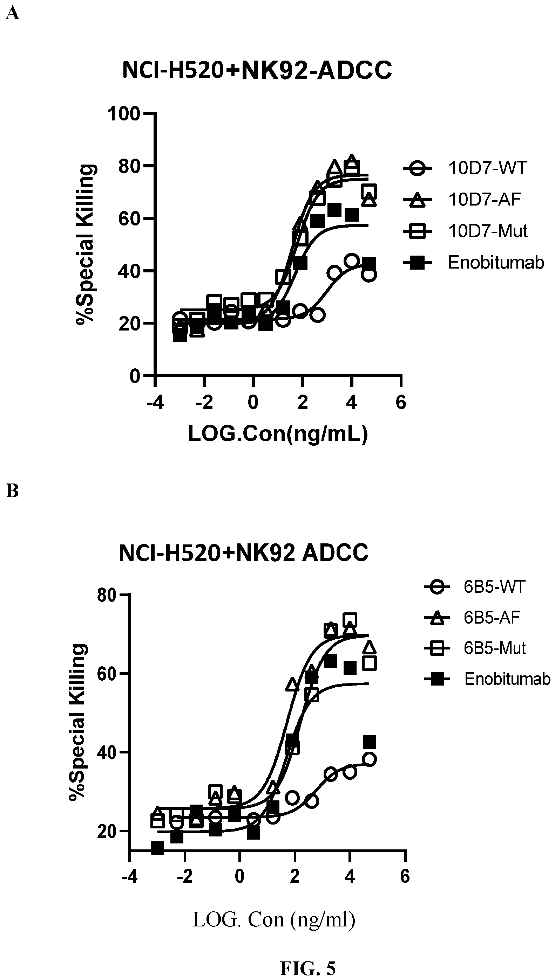

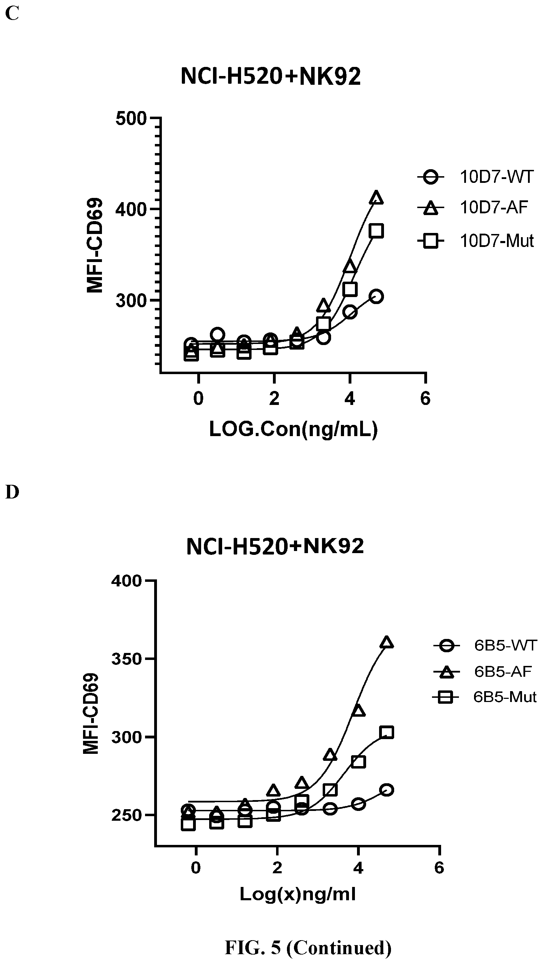

The anti-B7-H3 antibodies were further tested for their ability to induce human PBMC-mediated ADCC against NCI-H520 cells and HUVEC cells, wherein the NCI-H520 cells and the HUVEC cells were labelled using carboxyfluorescein succinimidyl ester (CFSE, Cat#: C34554I, Invitrogen). The human PBMCs were obtained by density gradient centrifugation using a lymphocyte separation medium, and cultured overnight in the medium (RIPM1640+10%FBS+300IU IL-2). The ADCC test was performed using the LIVE/DEAD fixable dead cell staining kit (Thermo Fisher, USA, Cat#: L34964). The target cells and the effector cells (PBMCs) were centrifuged at 1200 rpm for 5 min. The cells were re-suspended in the ADCC test medium (RIPM1640 medium + 1% FBS), and the cell viability was about 90% according to the cell counting results. Fifty µl of the target cells at the cell density of 4×10⁵/ml and 50 µl of the PBMCs at the cell density of 4×10⁶/ml were added to each well of the 96-well plates, the effector: target ratio being 10:1. The antibodies to be tested were added to each well, the starting final concentration was 50000 ng/ml and the other final concentrations were 5-fold diluted, 10 concentrations in total. The 96-well plates were incubated at 37°C for 24 hr, and the supernatant from each well was collected and measured for the IFN-γ concentration by ELISA (Cat#: SIF50, R&D) according to the manufacture's manual. The cells on the plates were washed with PBS for 3 times, and incubated with the LIVE/DEAD fixable dead cell stain at 37°C for 30 min. The cells were wash with PBS for 3 times, and subjected to FACS test. The cell death rate of the CFSE⁺ cells (NCI-H520 cells or HUVEC cells) was determined. Enoblituzumab was used as the positive control. The test results were shown in FIG. 5 to 8.

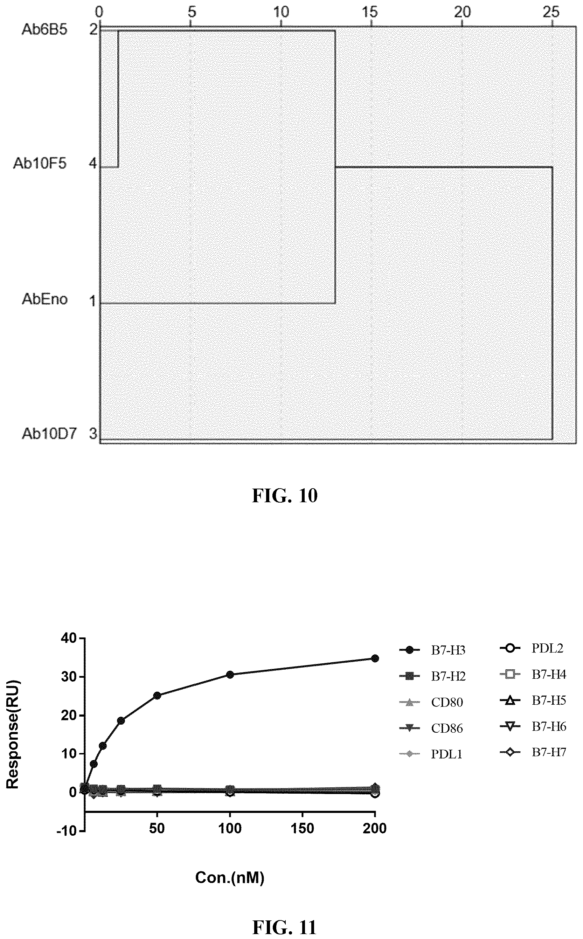

As shown in FIG. 5, 10D7-AF and 10D7-Mut had significantly higher ability than 10D7-WT to induce NK92-mediated ADCC against NCI-H520 tumor cells, and their ability was also higher than enoblituzumab, the positive control (A); 10D7-AF showed higher NK cell activation capability than 10D7-Mut, as it induced a higher level of the activation marker CD69 on the NK cell surfaces (C). Similarly, 6B5-AF and 6B5-Mut's ability to induce ADCC was significantly higher than 6B5-WT, and also higher than enoblituzumab, the positive control (B); in addition, 6B5-AF showed higher NK cell activation capability than 6B5-Mut, as it induced a higher level of CD69 (D). In addition, the positive control enoblituzumab showed an obvious hook effect in inducing NK92-mediated NCI-H520 killing at high doses, i.e., the killing effect as detected decreased when the antibody concentration increased after reaching a specified value, which effect was not observed in 10D7 or 6B5.

As shown in FIG. 6, the ability of 10D7-AF and 10D7-Mut to induce PBMC-mediated NCI-H520 tumor cell death was significantly higher than that of 10D7-WT, and also higher than that of the positive control enoblituzumab (A). Such trend was also observed for 6B5 in ADCC induction, i.e., the ADCC induced by 6B5-AF and 6B5-Mut against NCI-H520 tumor cells was more powerful than that of 6B5-WT, and also more powerful than that of the positive control enoblituzumab (B). Similarly, the positive control enoblituzumab showed an evident hook effect in inducing PBMC-mediated NCI-H520 death at high doses, which effect was not observed in 10D7 or 6B5.

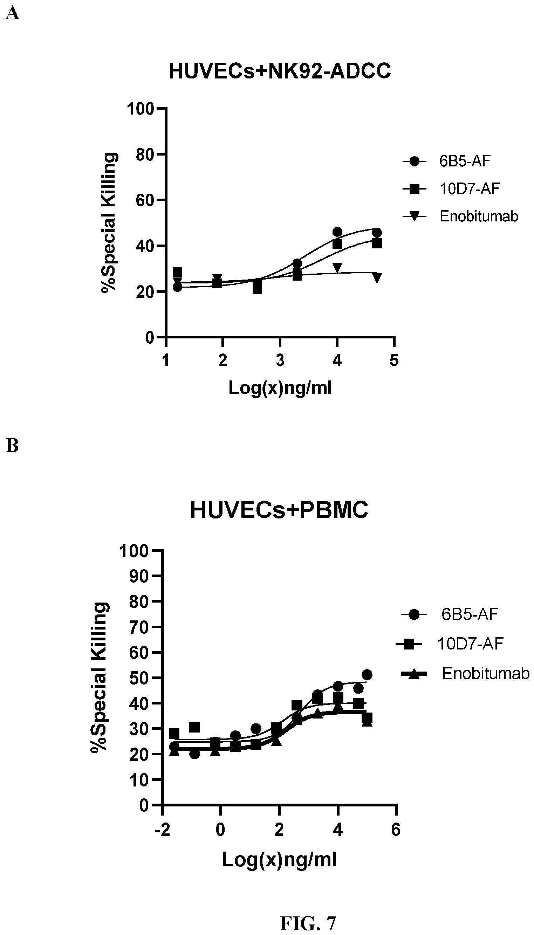

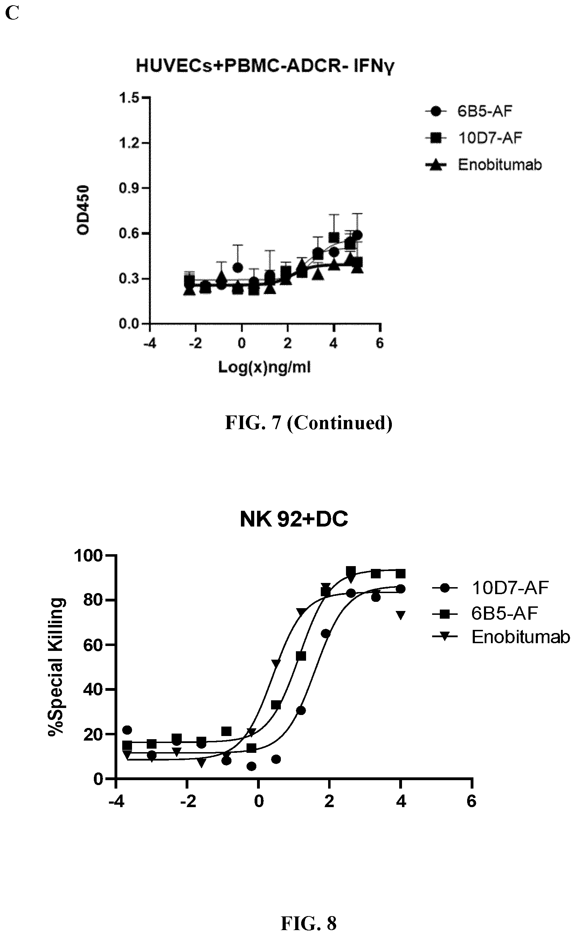

As shown in FIG. 7, 6B5-AF and 10D7-AF's ability to induce NK92-mediated HUVEC cell death was somewhat higher than enoblituzumab, the positive control (A), while in the reaction system involving PBMCs as the effector cells, none of 6B5-AF, 10D7-AF and enoblituzumab induced evident HUVEC cell death (B), or obvious IFN-γ release (C).

As shown in FIG. 8, the ability of 6B5-AF and 10D7-AF to induce DC killing by NK 92 cells was comparable to or lower than the positive control enoblituzumab.

### Example 6. Internalization of fully human anti-B7-H3 antibodies

To test whether the anti-B7-H3 antibodies of the disclosure can be internalized into cells, the A549 cells and the NCI-H520 cells were used for internalization, and enoblituzumab-IgG1-Mut and Omburtamab-IgG1 were used as the positive controls. Omburtamab was a radioactive iodine labeled anti-human B7-H3 monoclonal antibody developed by Y-mAbs Therapeutics, and Omburtamab-IgG1 contained the heavy and light chain variable regions of SEQ ID NOs: 41 and 42, the heavy chain constant region was a wildtype IgG1, and the light chain constant region was a κ constant region, respectively containing the amino acid sequences of SEQ ID NOs: 43 and 45.

The anti-human B7-H3 antibodies to be tested were evenly mixed with pHAb labelled goat-anti-human IgG1 Fc (Cat#: SSA015, Sino Biological, labelled by pHAb) at a 1:1 concentration ratio, which mixtures were mixed evenly with 10000 A549 cells, with the final concentrations of the anti-B7-H3 antibodies and the secondary antibodies both at 25 µg/mL, wherein the human IgG1 protein was used in the blank control wells. The mixtures were incubated in dark on ice for 1 hr, washed with centrifugation using pre-cooled FACS buffer (90% DMEM+10% FBS) for 3 times, with the supernatants discarded, added with pre-warmed complete culture medium, and co-incubated in a 37°C, 5% CO₂ incubator. The cell cultures were collected at 0, 3, 6, and 9 hr, and stored in dark on ice. After all samples were collected, they were centrifuged at low temperature at 1200 rpm for 3 min to discard the supernatants, and added and washed once with PBS buffer. The fluorescent signal from the cell surfaces was read. The raw data were collected from the flow cytometry for the cells incubated with different antibodies, and the MFI of the fluorescence emitted from the cells into which the different antibodies were internalized by binding to the cell surface B7-H3s were analyzed and determined based on the raw data. MFI curves were plotted based on the amount of the anti-B7-H3 antibodies as internalized.

Following the protocol above, the internalization of the anti-human B7-H3 antibodies was also tested using NCI-H520 cells.

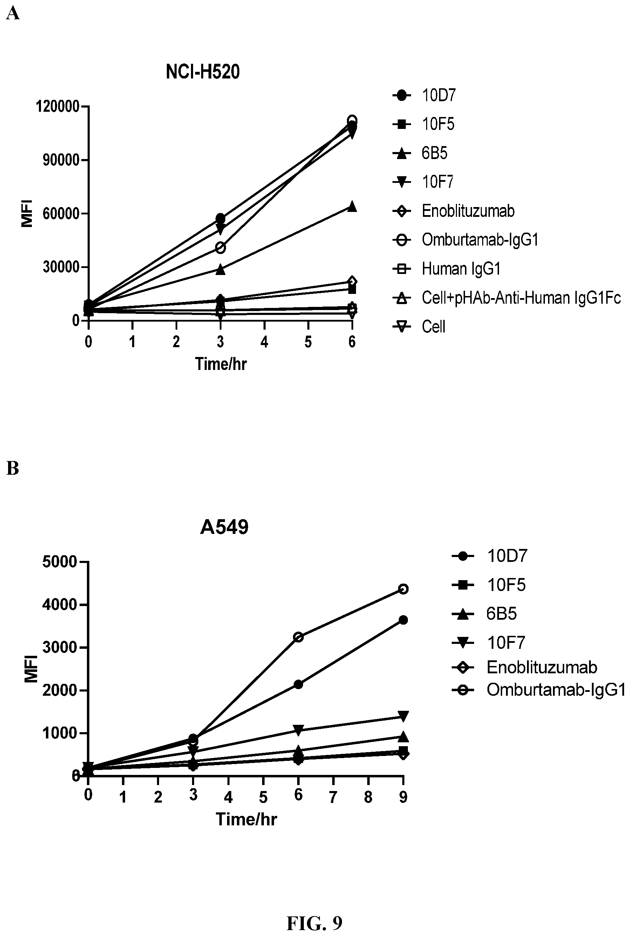

The results were shown in FIG. 9. During the whole test, no internalization occurred if only the cells, only cells plus secondary antibodies, or only cells, secondary antibodies and human IgG1 proteins were incubated, while MFI tended to increase as the time went when the cells were incubated with 6B5, 10D7, 10F5, 10F7, enoblituzumab or Omburtamab-IgG1. Especially, evident MFI elevation was observed for 10D7, 10F7 and Omburtamab-IgG1. The data indicated that 6B5, 10D7, 10F5, 10F7, enoblituzumab and Omburtamab-IgG1 were all able to be internalized by NCI-H520 and A549 cells, wherein the 10D7, 10F7 and Omburtamab-IgG1 were internalized at relatively high internalization rates.

### Example 7. Analysis of epitopes bound by fully human anti-B7-H3 antibodies

To determine the relationship among epitopes bound by the anti-human B7-H3 antibodies, each anti-human B7-H3 antibody was tested for its inhibitory effect on other antibodies' antigen binding using surface plasmon resonance (SPR) in an in-series manner. Four anti-human B7-H3 antibodies were tested in total, i.e., 6B5 ("Ab6B5"), 10D7 ("Ab10D7"), 10F5 ("Ab10F5") and enoblituzumab (briefly referred to as "AbEno").

The HBS-EP⁺ buffer (10 mM 4-(2-hydroethyl)-1-piperazine ethanesulfonic acid (HEPES), 150 mM NaCl, 3 mM ethylenediaminetetraacetic acid (EDTA) and 0.05% P20, pH7.4) was used as the running buffer. The anti-His antibodies were amine coupled to the surface of Series S sensor chip CM5, to generate the anti-His chip. Then, 1 M ethanolamine (pH8.5) was injected to block the remaining active carboxyl groups on the chip surface, and the chip was balanced with the HBS-EP⁺ buffer for 2 hr. The recombinant human B7-H3 (4Ig) proteins with His tag (Cat#: B7B-H52E7, Acrobiosystems) were injected into the Biacore 8K biosensor at the rate of 30 µL/min for 50 sec, and captured by the anti-His chip such that the protein density reached about 50 RU.

A pair of antibodies (concentration both at 800 nM) were injected to the chip in order at the rate of 30 µL/min. The binding time for the first injected antibody (Analyte 1) was 300 sec, and the binding time for the second antibodies as injected (Analyte 2) was 300 sec. During each analysis cycle, the chip was re-generated twice by using the glycine buffer (pH1.7, 30 µL/min, 30 sec) after antibody injection. The same test steps were performed for each pair of monoclonal antibodies, and each monoclonal antibody's inhibitory effect on antigen binding of another antibody was obtained. The binding values of each antibody were obtained using Biacore Insight. To quantify how one antibody interfered another antibody's antigen binding, the kinetic rates were determined for comparison of each pair of antibodies. Cluster analysis was performed using the statistics software.

As shown in FIG. 10, 4 anti-human B7-H3 antibodies were classified into 3 epitope clusters. In particular, 6B5 (Ab6B5) and 10F5 (Ab10F5) may bind the same the epitope, or their epitopes may overlap; 10D7 (Ab10D7) bound an unique epitope; the epitopes bound by 6B5 (Ab6B5), 10F5 (Ab10F5) and 10D7 (Ab10D7) and the epitope bound by enoblituzumab (AbEno) may overlap or be different.

### Example 8. Binding of anti-human B7-H3 antibodies to B7 family proteins

The anti-human B7-H3 antibodies were tested for their binding capability to B7 family proteins by SPR using Biacore T200 biosensor (Biacore, US) provided with a pre-fixed Protein A sensor chip.

In particular, the chip captured 1 µg/mL anti-human B7-H3 antibodies at the flow rate of 10 µL/min, and was injected for 180 sec at 30 µL/min with 200 nM, 100 nM, 50 nM, 25 nM, 12.5 nM, 6.25 nM or 0 nM recombinant human CD80 protein (human B7-1/CD80 protein, with His tag, Cat#: B71-H5228, Acrobiosystems), CD86 (human B7-2/CD86 protein, with His tag, Cat#: CD6-H5223, Acrobiosystems), PDL2 (human PD-L2/B7-DC protein, with His tag, Cat#: PD2-H5220, Acrobiosystems), PDL1 (human PD-L1B7-H1 protein, with His tag, Cat#: PD1-H5229, Acrobiosystems), B7-H2 (human B7-H2/ICOSLG protein, with His tag, Cat#: B72-H5221, Acrobiosystems), B7-H3 (4Ig) (Cat#: B7B-H52E7, Acrobiosystems), B7-H4 (human B7-H4 protein, with His tag (MALS verified), Cat#: BH7-HM174, Kactus Biosystems), B7-H5 (human B7-H5/Gi24/VISTA protein, with His tag (MALS verified), Cat#: B75-H52H0, Acrobiosystems), B7-H6 (human B7-H6/NCR3LG1 protein, with His tag, Cat#: B71-H5228, Acrobiosystems), and B7-H7 (human B7-H7/HHLA2 protein, with His tag (MALS verified), Cat#: B77-H52H5, Acrobiosystems), respectively. The dissociation was monitored for 600 sec. The data was analyzed using Biacore 8k evaluation software version 3.0, to obtain the binding responses, and the protein concentration-binding response curves were plotted, to determine the cross-reaction of the anti-human B7-H3 antibodies to the B7 family proteins.

The results were summarized in Table 4. Similar to enoblituzumab, the antibodies 6B5, 10D7 and 10F5 did not bind to the recombinant human CD80, CD86, PDL2, PDL1, B7-H2, B7-H3, B7-H4, B7-H5, B7-H6 or B7-H7 proteins, but specifically bound to the recombinant human B7-H3 protein. The results of 6B5's binding to the recombinant human proteins were shown in FIG. 11. The binding responses of each antibody to the recombinant human proteins at 200 nM were summarized in Table 4 below.

**Table 4. Binding of fully human anti-B7-H3 antibodies to B7 family proteins**

| Antibody | Response (RU) | | | | | | | | | |
|---|---|---|---|---|---|---|---|---|---|---|
| | B7-H3 | B7-H2 | CD80 | CD86 | PDL1 | PDL2 | B7-H4 | B7-H5 | B7-H6 | B7-H7 |
| 6B5 | 34.8 | 0.9 | 0.6 | 0.3 | 1 | -0.2 | 0.5 | 0.9 | -0.2 | 1.3 |
| 10D7 | 41.9 | -0.1 | 0.2 | -0.2 | 0.3 | -0.2 | 0.3 | 0.6 | -0.5 | 0.2 |
| 10F5 | 32.5 | 0.9 | 0.7 | 0.5 | 0.5 | 0 | 0.3 | 0.6 | -0.4 | 1.7 |
| enoblituzumab | 25.7 | -2.0 | -1.8 | -1.5 | -2.2 | -1.2 | -2.0 | -1.8 | -2.2 | -1.3 |

### Example 9. In vivo anti-tumor effect of fully human anti-B7-H3 antibodies

The antibodies 6B5, 10D7, 10F5 and the positive control enoblituzumab were tested for their *in vivo* anti-tumor activity. To better show the biological effect of the antibody variable regions, all the antibodies chose human IgG1 as the Fc region which was introduced with 5 point mutations (L235V, F243L, R292P, Y300L and P396L, according to EU numbering, SEQ ID NO: 44) to enhance ADCC.

In the first experiment, the mice with human B7-H3 gene (Cat#: 110028, Biocytogen) were subcutaneously injected with 2× 10⁵ EL4 cells over-expressing human B7-H3 proteins at one side of the abdomen. When the tumors reached 90-120 mm³, the mice were randomly allocated into 3 groups, (n=8/group), and intraperitoneally injected with PBS (G1), enoblituzumab (G2), and 10F5-Mut (G3), respectively, at the dose of 10 mg/kg, twice a week, 6 doses in total. The day doing the grouping was designated as Day 0, and administration began on this day.

In another experiment, the B7-H3 humanized mice were randomly allocated into 4 groups (n=8/group) when tumors grew to 90-120 mm³, and intraperitoneally injected with PBS (G1), enoblituzumab (G2), 6B5-Mut (G3) and 15F11-Mut (G4), respectively, with the same dose and the same dosing frequency as above. The day doing the grouping was designated as Day 0, and administration began on this day.

During the test, the mice were weighed. The tumor sizes were measured twice a week, and the tumor size was calculated as V=(length × width²)/2. The tumor growth inhibition was calculated as TGI (%)=[1 - (Ti - T₀)/(Vi - V₀)] × 100, wherein Tᵢ referred to the average tumor volume of a treatment group on Day i, and T₀ was the average tumor volume of that treatment group on Day 0, Vᵢ was the average tumor volume of the control group on Day i, and V₀ was the average tumor volume of the control group on Day 0.

During the whole test, each group of animals survived with a good heath status. As compared to the control group, the mice of the treatment groups showed no evident body weight change (P>0.05). In particular, the average body weight of each group was 19.3-19.5 on Day 0 when grouping was done, and the average body weight was 19.8-20.9 when the test ended (Day 17 after grouping for the first experiment, and Day 20 after grouping for the second experiment), so the body weight change was between 103.2% and 111.1%. The results showed that the anti-human B7-H3 antibodies as tested were well tolerated by the mice, i.e., all antibodies were non-toxic to the mice.

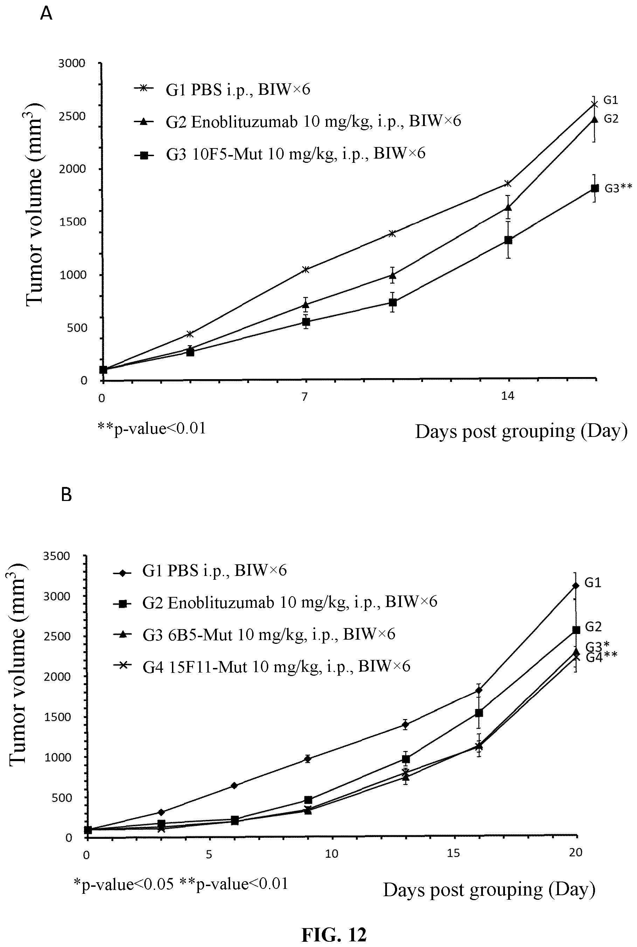

FIG. 12 showed the tumor size change of the mice from each group. It can be seen that, as compared to the control group, all the anti-human B7-H3 antibodies slowed down or inhibited tumor growth to some extent. Particularly, 10F5-Mut (A), 6B5-Mut and 15F11-Mut (B) showed higher anti-tumor effect than the positive control enoblituzumab.

The main data and analysis results were set forth in Table 5 and Table 6, including the tumor sizes on the grouping day and 7 or 14 days after grouping, the tumor volume at the end of the experiment, mouse survival rate, tumor (volume) inhibition rate (TGI_{TV}%), and the statistical significance between the treatment group(s) and the control group (P value). The data showed that 6B5-Mut, 15F11-Mut and 10F5-Mut all significantly inhibited tumor growth, with 10F5-Mut's anti-tumor effect being the best and 15F11-Mut's being the second best.

**Table 5. Summary of in vivo anti-tumor effect of fully human anti-human B7-H3 antibody 10F5-Mut**

| | Tumor size (mm³) | | | | Survi val | TGI_{TV} % | P value | |
|---|---|---|---|---|---|---|---|---|
| | Day 0 | Day 7 | Day 14 | Day 17 | | | Body weight | Tumor size |
| PBS | 104 ± 4 | 1038± 69 | 1838 ± 75 | 2584± 91 | 8/8 | n.a. | n.a. | n.a. |
| enoblituzumab | 104 ± 5 | 706 ± 66 | 1614 ± 110 | 2443± 217 | 8/8 | 5.7% | 0.003 | 0.560 |
| 10F5-Mut | 104 ± 4 | 544 ± 65 | 1306 ± 172 | 1790 ± 131 | 8/8 | 32.0% | 0.176 | 2.021E-04 |

**Table 6. Summary of in vivo anti-tumor effect of fully human anti-human B7-H3 antibodies 6B5-Mut and 15F11-Mut**

| | Tumor size (mm³) | | | | Survival | TGI_{TV}% | P value | |
|---|---|---|---|---|---|---|---|---|
| | Day 0 | Day 6 | Day 13 | Day 20 | | | Body weight | Tumor size |
| PBS | 98 ± 3 | 637 ± 19 | 1383 ± 64 | 3093 ± 164 | 8/8 | n.a. | n.a. | n.a. |
| enoblituzumab | 98 ± 3 | 219 ± 30 | 957 ± 90 | 2544 ± 374 | 8/8 | 18.3% | 0.029 | 0.200 |
| 6B5-Mut | 98 ± 3 | 191 ± 16 | 730 ± 94 | 2277 ± 261 | 8/8 | 27.3% | 0.083 | 0.019 |
| 15F11-Mut | 98 ± 3 | 189 ± 19 | 788 ± 50 | 2206 ± 127 | 8/8 | 29.6% | 0.192 | 0.001 |

### Example 10. Cross-reactive staining of normal human tissues by fully human anti-human B7-H3 antibody

The fully human anti-human B7-H3 antibody 6B5-AF was tested for its cross-reaction to 35 kinds of frozen normal human tissues (each tissue from 3 individuals) by immunohistochemical staining utilizing streptavidin-biotin binding, with enoblituzumab used as the control antibody. The human body tissues were listed in Table 7.

The immunohistochemical staining was done as follows. The frozen sections were dried at ambient temperature, dipped in PBST for 5-10 min; added with 0.3% hydrogen peroxide/anhydrous methanol, allowed to react at ambient temperature for 10-15 min, dipped in PBST for 3 times; added and reacted with avidin at ambient temperature for 10-15 min, dipped in PBST for 3 times; added and reacted with d-biotin solution at ambient temperature for 10-15 min, dipped in PBST for 3 times; added with sheep serum (working solution) for blocking, allowed to react at ambient temperature for 10-15 min, solutions discarded; added with primary antibodies (enoblituzumab-biotin, 6B5-AF-biotin, human IgG1/κ isotype control-biotin) or PBST, reacted at ambient temperature for 10-15 min, dipped in PBST for 3 times; added with streptavidin-peroxidase, incubated at ambient temperature for 10-15 min, dipped in PBST for 3 times; color developed with DAB, counterstained with hematoxylin, dehydrated in alcohol with increasing concentrations and then xylene; added with medium, covered with cover slips, air dried for microscopic examination.

**Table 7. 35 kinds of frozen normal human tissues**

| | | | | |
|---|---|---|---|---|
| adrenal gland | aorta | bladder | blood cells | marrow |
| mammary gland | cerebellum | cerebrum | colon | duodenum |
| eye | esophagus | oviduct | stomach | heart |
| kidney | liver | lung | lymph node | ovary |
| pancreas | parathyroid gland | pituitary | placenta | prostate |
| skin | spinal cord | spleen | skeletal muscle | testis |
| thymus | thyroid | ureter | cervix | endometrium |

The staining results were observed via the microscope, and the levels of the antibodies bound to the tissues were determined by the staining. According to the staining intensity and the percent of the cell as stained, the staining results were scored (positive cell staining intensity: 0=no cell staining; 1= weak or vague staining; 2=weak positive staining; 3=intermediate positive staining; 4=strong positive staining; M=tissue missing or not applicable. Proportion of positive cells in cells of the same type: 0=no cell staining; 1=positive cells accounting for less than 25% of the cells of the same type; 2= positive cells accounting for 25%-50% of the cells of the same type; 3= positive cells accounting for 50%-75% of the cells of the same type; 4= positive cells accounting for more than 75% of the cells of the same type; M=tissue missing or not applicable). The tissues without any positive staining from the primary antibodies (enoblituzumab-biotin, 6B5-AF-biotin, human IgG1/κ isotype control-biotin) or PBST were excluded from the statistics, and the scores of the tissues with positive staining were summarized in Table 8.

It can be seen from the human tissue staining results in Table 8 that, the tissue staining specificity of the fully human anti-B7-H3 antibody 6B5-AF was consistent with that of enoblituzumab. That was, among the 35 kinds of normal human tissues as tested, 24 normal human tissues got negative staining results, and only 11 normal tissues got weak staining. In the meanwhile, as the B7-H3 antigen epitope difference between 6B5-AF and enoblituzumab, the staining intensity was a bit different in some tissues having positive staining. As shown in Table 8, the staining in the pituitary was relatively stronger while the staining in thymus, cervix and endometrium was a bit weak for 6B5-AF, as compared to enoblituzumab.

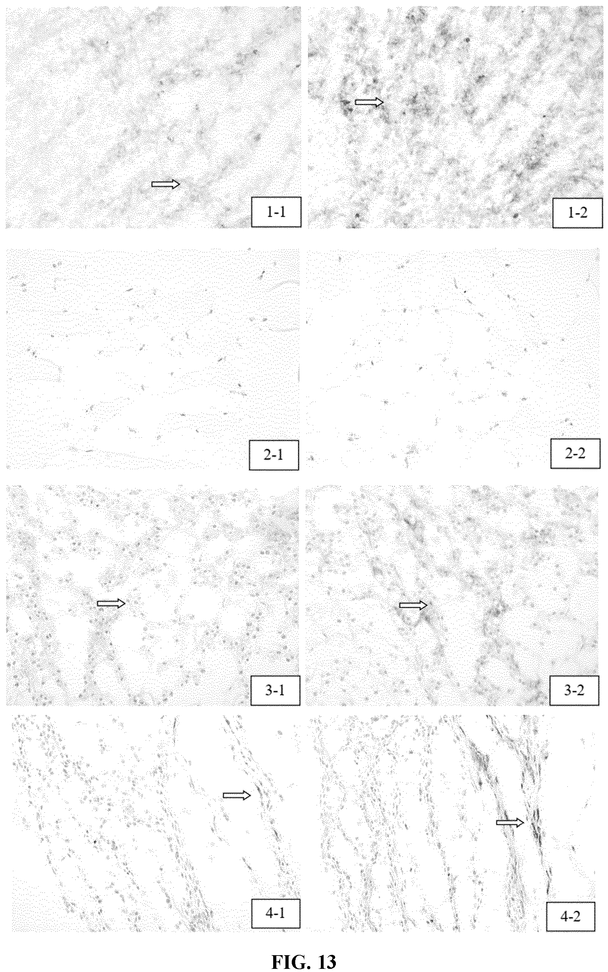

FIG. 13 showed the staining results of representative tissues, wherein 1-1, 2-1, 3-1 and 4-1 showed enoblituzumab's staining in spleen, skeletal muscle, adrenal gland and bladder, while 1-2, 2-2, 3-2 and 4-2 showed 6B5-AF's staining in spleen, skeletal muscle, adrenal gland and bladder.

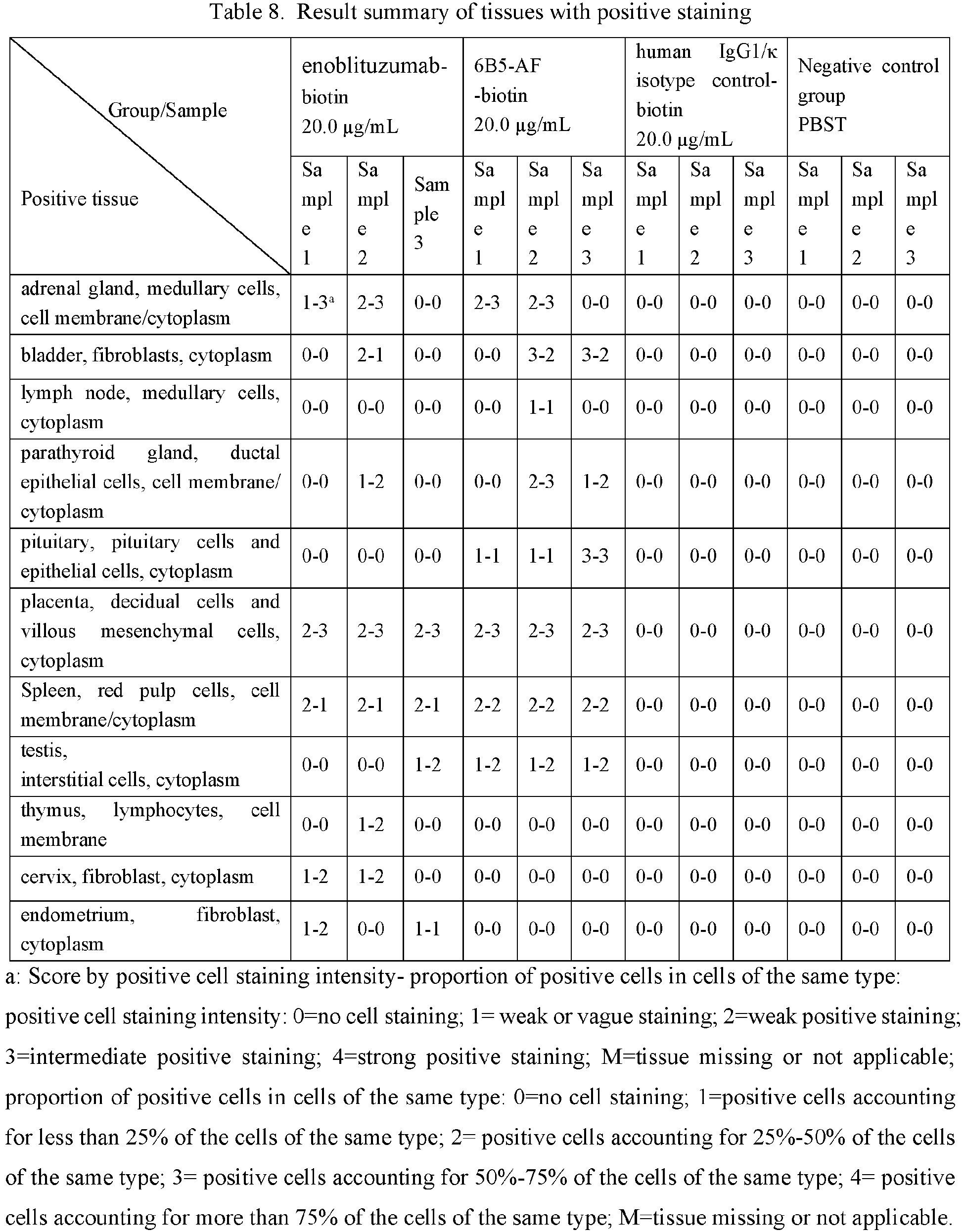

Sequences mentioned in the present application are summarized in Table 9.

**Table 9. Sequences**

| Description/Sequence/SEQ ID NO. |
|---|
| VH-CDR1 of 6B5 |
| SGGYYWS (SEQ ID NO: 1) |
| VH-CDR2 of 6B5 |
| YIYYSGGTYYSPSLKS (SEQ ID NO: 2) |
| VH-CDR3 of 6B5 |
| RAGYSSSFDS (SEQ ID NO: 3) |
| VL-CDR1 of 6B5 |
| RSSQSLLYSNGYTYLD (SEQ ID NO: 4) |
| VL-CDR2 of 6B5 |
| LGFNRAS (SEQ ID NO: 5) |
| VL-CDR3 of 6B5 |
| MQGLQTPYT (SEQ ID NO: 6) |
| VH of 6B5 |
| QVQLVESGPGLVKPSQTLSLTCTVSDGSISSGGYYWSWIRQHPGKGLEWIGYIYYSGGTY YSPSLKSRVTISVDTSKNQFSLKLTSVTAADTAVYYCARRAGYSSSFDSWGQGILVTVSS (SEQ ID NO: 29) |
| VL of 6B5 |
| EIVLTQSPLSLPVTPGEPASISCRSSQSLLYSNGYTYLDWYLQKPGQSPQFLIYLGFNRASG VPDRFSGSGSGTDFTLKISRVEAEDVGVYYCMQGLQTPYTFGQGTKLEIK (SEQ ID NO: 30) |
| VH-CDR1 of 15F11 |
| SGGYYWS (SEQ ID NO: 1) |
| VH-CDR2 of 15F11 |
| YIYYSGSTYYNPSLKS (SEQ ID NO: 7) |
| VH-CDR3 of 15F11 |
| ERIAVAGYFDY (SEQ ID NO: 8) |
| VL-CDR1 of 15F11 |
| RASQSVSSSYLA (SEQ ID NO: 9) |
| VL-CDR2 of 15F11 |
| GASTRAT (SEQ ID NO: 10) |
| VL-CDR3 of 15F11 |
| QQYGSSPLT (SEQ ID NO: 11) |
| VH of 15F11 |
| EVQLVQSGPGLVKPSQTLSLTCTVSGGSINSGGYYWSWIRQHPGKGLEWIGYIYYSGSTY YNPSLKSRVTISIDTSKNQFSLKLSSVTAADTAVYYCARERIAVAGYFDYWGQGTLVTVSS (SEQ ID NO: 31) |
| VL of 15F11 |
| EIVMTQSPGTLSLSPGESATLSCRASQSVSSSYLAWYQQKPGQAPRLLIYGASTRATGIPDR FSGSGSGTDFTLTISRLEPEDFAVYFCQQYGSSPLTFGGETKVEIK (SEQ ID NO: 32) |
| VH-CDR1 of 10D7 |
| RSSYYWG (SEQ ID NO: 12) |
| VH-CDR2 of 10D7 |
| NIFYAGSTYYNPSLKS (SEQ ID NO: 13) |
| VH-CDR3 of 10D7 |
| RITGTTGWFDP (SEQ ID NO: 14) |
| VL-CDR1 of 10D7 |
| RASQGIRNDLG (SEQ ID NO: 15) |
| VL-CDR2 of 10D7 |
| AASTLQS (SEQ ID NO: 16) |
| VL-CDR3 of 10D7 |
| LQDYNYPRT (SEQ ID NO: 17) |
| VH of 10D7 |
| QVQLVQSGPGLVKPSETLSLTCTVSGGSISRSSYYWGWIRQPPGKGLEWIGNIFYAGSTYY NPSLKSRVTISVDTSKNQFSLKLSSVTAADTAVYYCARRITGTTGWFDPWGQGTLVTVSS (SEQ ID NO: 33) |
| VL of 10D7 |
| DIQLTQSPSSLSASVGDRVTITCRASQGIRNDLGWYQQKPGKAPKLLIYAASTLQSGVPSRF SGSGSGTDFTLTISSLQPEDFATYYCLQDYNYPRTFGQGTKVEIK (SEQ ID NO: 34) |
| VH-CDR1 of 10F7 |
| SSSAAWN (SEQ ID NO: 18) |
| VH-CDR2 of 10F7 |
| RTYYRSKWYNDFAVSVKS (SEQ ID NO: 19) |
| VH-CDR3 of 10F7 |
| DLITLVRGVILPFDY (SEQ ID NO: 20) |
| VL-CDR1 of 10F7 |
| RASQSVSSSYLA (SEQ ID NO: 9) |
| VL-CDR2 of 10F7 |
| GASSRAT (SEQ ID NO: 21) |
| VL-CDR3 of 10F7 |
| QQYGSSHT (SEQ ID NO: 22) |
| VH of 10F7 |
| QVQLVQSGPGLVKPSQTLSLTCAISGDSVSSSSAAWNWIRQSPSRGLEWLGRTYYRSKWY NDFAVSVKSRITINPDTSKNQFSLQLNSVTPEDTAVYYCARDLITLVRGVILPFDYWGQGT LVTVSS (SEQ ID NO: 35) |
| VL of 10F7 |
| EIVLTQSPGTLSLSPGERATLSCRASQSVSSSYLAWYQQRPGQAPRLLIYGASSRATGIPDR FSGSGSGTDFTLTISRLEPEDFAVYYCQQYGSSHTFGQGTNLEIK (SEQ ID NO: 36) |
| VH-CDR1 of 10F5 |
| SQAIS (SEQ ID NO: 23) |
| VH-CDR2 of 10F5 |
| RIIPMLGIGDYAQSFQG (SEQ ID NO: 24) |
| VH-CDR3 of 10F5 |
| EGIAALGTNYYYGMDV (SEQ ID NO: 25) |
| VL-CDR1 of 10F5 |
| RASQGISRWLA (SEQ ID NO: 26) |
| VL-CDR2 of 10F5 |
| VASSLQS (SEQ ID NO: 27) |
| VL-CDR3 of 10F5 |
| QQANSFPFT (SEQ ID NO: 28) |
| VH of 10F5 |
| QVQLVQSGAEVKKPGSSVKVSCKASGGTFSSQAISWVRQAPGQGLEWMGRIIPMLGIGDY AQSFQGRVTITADKSTSTAYLEVSSLGSEDTAVYYCAREGIAALGTNYYYGMDVWGQGT AVTVSS (SEQ ID NO: 37) |
| VL of 10F5 |
| EIVMTQSPSSVSASVGDRVTITCRASQGISRWLAWYQQKPGKAPKLLIYVASSLQSGVPSR FSGSGSGTDFTLTISSLQPEDFATYYCQQANSFPFTFGPGTKVDIR (SEQ ID NO: 38) |
| VH of enoblituzumab |
| EVQLVESGGGLVQPGGSLRLSCAASGFTFSSFGMHWVRQAPGKGLEWVAYISSDSSAIYY ADTVKGRFTISRDNAKNSLYLQMNSLRDEDTAVYYCGRGRENIYYGSRLDYWGQGTTVT VSS (SEQ ID NO: 39) |
| VL of enoblituzumab |
| DIQLTQSPSFLSASVGDRVTITCKASQNVDTNVAWYQQKPGKAPKALIYSASYRYSGVPSR FSGSGSGTDFTLTISSLQPEDFATYYCQQYNNYPFTFGQGTKLEIK (SEQ ID NO: 40) |
| VH of omburtamab |
| QVQLQQSGAELVKPGASVKLSCKASGYTFTNYDINWVRQRPEQGLEWIGWIFPGDGSTQ YNEKFKGKATLTTDTSSSTAYMQLSRLTSEDSAVYFCARQTTATWFAYWGQGTLVTVSA (SEQ ID NO: 41) |
| VL of omburtamab |
| DIVMTQSPATLSVTPGDRVSLSCRASQSISDYLHWYQQKSHESPRLLIKYASQSISGIPSRFS GSGSGSDFTLSINSVEPEDVGVYYCQNGHSFPLTFGAGTKLELK (SEQ ID NO: 42) |
| human IgG1 constant region (wildtype) |
| ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSREEMT |
| KNQVSLTCLVKGFYPSDIAVEWESNGQPENNYKTTPPVLDSDGSFFLYSKLTVDKSRWQQ GNVFSCSVMHEALHNHYTQKSLSLSPGK (SEQ ID NO: 43) |
| human IgG1 constant region (human IgG1-Mut, L235V, F243L, R292P, Y300L and P396L, according to EU numbering) |
| ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKRVEPKSCDKTHTCPPCPAPELVGGPS VFLLPPKPKDTLMISRTPEVTCVVVDVSHEDPEVKFNWYVDGVEVHNAKTKPPEEQYNST LRVVSVLTVLHQDWLNGKEYKCKVSNKALPAPIEKTISKAKGQPREPQVYTLPPSREEMT KNQVSLTCLVKGFYPSDIAVEWESNGQPENNYKTTPLVLDSDGSFFLYSKLTVDKSRWQQ GNVFSCSVMHEALHNHYTQKSLSLSPGK (SEQ ID NO: 44) |
| human κ constant region |
|  (SEQ ID NO: 45) |
| Hereinafter list the CDRs defined by Chothia |
| VH-CDR1 of 6B5 |
| DGSISSGGY (SEQ ID NO: 46) |
| VH-CDR2 of 6B5 |
| YYSGG (SEQ ID NO: 47) |
| VH-CDR3 of 6B5 |
| RAGYSSSFDS (SEQ ID NO: 48) |
| VL-CDR1 of 6B5 |
| RSSQSLLYSNGYTYLD (SEQ ID NO: 49) |
| VL-CDR2 of 6B5 |
| LGFNRAS (SEQ ID NO: 50) |
| VL-CDR3 of 6B5 |
| MQGLQTPYT (SEQ ID NO: 51) |
| VH-CDR1 of 15F11 |
| GGSINSGGY (SEQ ID NO: 52) |
| VH-CDR2 of 15F11 |
| YYSGS (SEQ ID NO: 53) |
| VH-CDR3 of 15F11 |
| ERIAVAGYFDY (SEQ ID NO: 54) |
| VL-CDR1 of 15F11 |
| RASQSVSSSYLA(SEQ ID NO: 55) |
| VL-CDR2 of 15F11 |
| GASTRAT (SEQ ID NO: 56) |
| VL-CDR3 of 15F11 |
| QQYGSSPLT (SEQ ID NO: 57) |
| VH-CDR1 of 10D7 |
| GGSISRSSY (SEQ ID NO: 58) |
| VH-CDR2 of 10D7 |
| FYAGS (SEQ ID NO: 59) |
| VH-CDR3 of 10D7 |
| RITGTTGWFDP (SEQ ID NO: 60) |
| VL-CDR1 of 10D7 |
| RASQGIRNDLG (SEQ ID NO: 61) |
| VL-CDR2 of 10D7 |
| AASTLQS (SEQ ID NO: 62) |
| VL-CDR3 of 10D7 |
| LQDYNYPRT (SEQ ID NO: 63) |
| VH-CDR1 of 10F7 |
| GDSVSSSSA (SEQ ID NO: 64) |
| VH-CDR2 of 10F7 |
| YYRSKWY (SEQ ID NO: 65) |
| VH-CDR3 of 10F7 |
| DLITLVRGVILPFDY (SEQ ID NO: 66) |
| VL-CDR1 of 10F7 |
| RASQSVSSSYLA (SEQ ID NO: 67) |
| VL-CDR2 of 10F7 |
| GASSRAT (SEQ ID NO: 68) |
| VL-CDR3 of 10F7 |
| QQYGSSHT (SEQ ID NO: 69) |
| VH-CDR1 of 10F5 |
| GGTFSSQ (SEQ ID NO:70) |
| VH-CDR2 of 10F5 |
| IPMLGI (SEQ ID NO: 71) |
| VH-CDR3 of 10F5 |
| EGIAALGTNYYYGMDV (SEQ ID NO: 72) |
| VL-CDR1 of 10F5 |
| RASQGISRWLA (SEQ ID NO: 73) |
| VL-CDR2 of 10F5 |
| VASSLQS (SEQ ID NO: 74) |
| VL-CDR3 of 10F5 |
| QQANSFPFT (SEQ ID NO: 75) |

Although the invention has been described with one or more embodiments, it is to be understood that the invention is not to be limited to these embodiments, and the description above is intended to encompass all other alternatives, modifications and equivalents encompassed within the spirit and scope of the accompanying claims. All references cited herein are incorporated by reference in their entirety.

## Claims

1. An isolated monoclonal antibody or an antigen binding portion thereof, binding to B7-H3, comprising
i) a heavy chain variable region, wherein the heavy chain variable region comprises a VH CDR1 region, a VH CDR2 region and a VH CDR3 region, wherein the VH CDR1 region, the VH CDR2 region and the VH CDR3 region comprise amino acid sequences having at least 80%, 85%, 90%, 91%, 92%, 93%, 94%, 95%, 96%, 97%, 98%, 99% or 100% identity to (1) SEQ ID NOs: 1, 2 and 3, respectively; (2) SEQ ID NOs: 1, 7 and 8, respectively; (3) SEQ ID NOs: 12, 13 and 14, respectively; (4) SEQ ID NOs: 18, 19 and 20, respectively; or (5) SEQ ID NOs: 23, 24 and 25, respectively; and/or
ii) a light chain variable region, wherein the light chain variable region comprises a VL CDR1 region, a VL CDR2 region and a VL CDR3 region, wherein the VL CDR1 region, the VL CDR2 region, and the VL CDR3 region comprise amino acid sequences having at least 80%, 85%, 90%, 91%, 92%, 93%, 94%, 95%, 96%, 97%, 98%, 99% or 100% identity to (1) SEQ ID NOs: 4, 5 and 6, respectively; (2) SEQ ID NOs: 9, 10 and 11, respectively; (3) SEQ ID NOs: 15, 16 and 17, respectively; (4) SEQ ID NOs: 9, 21 and 22, respectively; or (5) SEQ ID NOs: 26, 27 and 28, respectively.

2. The isolated monoclonal antibody or the antigen binding portion thereof of claim 1, wherein the heavy chain variable region comprises an amino acid sequence having at least 80%, 85%, 90%, 91%, 92%, 93%, 94%, 95%, 96%, 97%, 98%, 99% or 100% identity to SEQ ID NOs: 29, 31, 33, 35 or 37.

3. The isolated monoclonal antibody or the antigen binding portion thereof of claim 1, wherein the light chain variable region comprises an amino acid sequence having at least 80%, 85%, 90%, 91%, 92%, 93%, 94%, 95%, 96%, 97%, 98%, 99% or 100% identity to SEQ ID NOs: 30, 32, 34, 36 or 38.

4. The isolated monoclonal antibody or the antigen binding portion thereof of claim 2, wherein the heavy chain variable region and the light chain variable region comprise amino acid sequences having at least 80%, 85%, 90%, 91%, 92%, 93%, 94%, 95%, 96%, 97%, 98%, 99% or 100% identity to (1) SEQ ID NOs: 29 and 30, respectively; (2) SEQ ID NOs: 31 and 32, respectively; (3) SEQ ID NOs: 33 and 34, respectively; (4) SEQ ID NOs: 35 and 36, respectively; or (5) SEQ ID NOs: 37 and 38, respectively.

5. An isolated monoclonal antibody or an antigen binding portion thereof, binding to B7-H3, comprising
i) a heavy chain variable region, wherein the heavy chain variable region comprises a VH CDR1 region, a VH CDR2 region and a VH CDR3 region, wherein the VH CDR1 region, the VH CDR2 region and the VH CDR3 region have identity to a VH CDR1 region, a VH CDR2 region and a VH CDR3 region of a specified VH sequence, respectively; and/or
ii) a light chain variable region, wherein the light chain variable region comprises a VL CDR1 region, a VL CDR2 region and a VL CDR3 region, wherein the VL CDR1 region, the VL CDR2 region, and the VL CDR3 region have identity to a VL CDR1 region, a VL CDR2 region and a VL CDR3 region of a specified VL sequence, respectively,
wherein the specified VH sequence and the specified VL sequence are selected from the group consisting of
(1) the specified VH sequence and the specified VL sequence set forth in SEQ ID NOs: 29 and 30, respectively;
(2) the specified VH sequence and the specified VL sequence set forth in SEQ ID NOs: 31 and 32, respectively;
(3) the specified VH sequence and the specified VL sequence set forth in SEQ ID NOs: 33 and 34, respectively;
(4) the specified VH sequence and the specified VL sequence set forth in SEQ ID NOs: 35 and 36, respectively;
(5) the specified VH sequence and the specified VL sequence set forth in SEQ ID NOs: 37 and 38, respectively.

6. The isolated monoclonal antibody or the antigen binding portion thereof of any one of claims 1 to 5, comprising a heavy chain constant region and a light chain constant region, wherein the heavy chain constant region is a human IgG1 constant region, and the light chain constant region is a human κ constant region.

7. The isolated monoclonal antibody or the antigen binding portion thereof of claim 6, wherein the heavy chain constant region comprises the amino acid sequence of SEQ ID NO: 44, and the light chain constant region comprises the amino acid sequence of SEQ ID NO: 45.

8. The isolated monoclonal antibody or the antigen binding portion thereof of any one of claims 1 to 7, which is an afucosylated monoclonal antibody or an antigen binding portion thereof.

9. The isolated monoclonal antibody or the antigen binding portion thereof of any one of claims 1 to 8, which (a) binds to human B7-H3, (b) binds to monkey B7-H3, (c) binds to mouse B7-H3, (d) binds to B7-H3⁺ cells, (e) is able to activate immune cells, (f) is able to induce ADCC against B7-H3⁺ cells, and/or (g) has *in vivo* anti-tumor effect.

10. A bispecific molecule, an immunoconjugate, a chimeric antigen receptor, an engineered T cell receptor, or an oncolytic virus, comprising the isolated monoclonal antibody or the antigen binding portion thereof of any one of claims 1 to 9.

11. A nucleic acid molecule encoding the isolated monoclonal antibody or the antigen binding portion thereof of any one of claims 1 to 9.

12. A pharmaceutical composition, comprising the isolated monoclonal antibody or the antigen binding portion thereof of any one of claims 1 to 9, and a pharmaceutically acceptable carrier.

13. Use of the isolated monoclonal antibody or the antigen binding portion thereof of any one of claims 1 to 9, or the pharmaceutical composition of claim 11 in preparation of a medicament for treating a B7-H3 associated disease.

14. The use of claim 13, wherein the B7-H3 associated disease is a tumor.

15. The use of claim 14, wherein the tumor is selected from the group consisting of breast cancer, melanoma, prostate cancer, head and neck squamous cell carcinoma, lung cancer, non-small cell lung cancer, central nervous system neuroblastoma, glioblastoma, desmoplastic small round cell tumor, diffuse pontine glioma, medulloblastoma, pancreatic cancer, liver cancer, colorectal cancer, non-Hodgkin lymphoma, esophageal cancer, ovarian cancer, bladder cancer, small cell carcinoma, endometrial cancer, kidney cancer, gastric cancer, acute myeloid leukemia, hepatocellular carcinoma, hypopharyngeal squamous cell carcinoma, urothelial cell carcinoma.

16. Use of the isolated monoclonal antibody or the antigen binding portion thereof of any one of claims 1 to 9, or the pharmaceutical composition of claim 11 in preparation of a medicament for enhancing immune responses.
